# EUROPÄISCHE PATENTSCHRIFT

(11) **EP 2 247 287 B1**
(45) Veröffentlichungstag und Bekanntmachung des Hinweises auf die Patenterteilung: **12.08.2015**
(21) Anmeldenummer: 09705184.1
(22) Anmeldetag: 29.01.2009
(51) Int. Cl.: A61K 31/00, A61K 31/739, A61K 31/522, A61K 45/06, G01N 33/68, A61P 25/28

(54) **Verfahren zur Identifizierung von therapiebedürftigen Patienten mit leichten kognitiven Störungen und die Behandlung derartiger Patienten**
Method for the identification of patients in need of therapy having minor cognitive disorders and the treatment of such patients
Procédé d'identification de patients présentant de légers troubles cognitifs, nécessitant un traitement, et traitement de ces patients

(30) Priorität: 01.02.2008 DE 102008007218; 27.07.2008 EP 08161228
(43) Veröffentlichungstag der Anmeldung: 10.11.2010
(73) Patentinhaber: B.R.A.H.M.S GmbH, 16761 Hennigsdorf (DE)
(72) Erfinder: BERGMANN, Andreas, 12351 Berlin (DE)
(74) Vertreter: Kilger, Ute
(86) Internationale Anmeldenummer: PCT/EP2009/051035
(87) Internationale Veröffentlichungsnummer: WO 2009/095454

(56) Entgegenhaltungen:
- WO-A-89/00428
- WO-A-03/045950
- WO-A-03/097062
- WO-A-2004/082455
- WO-A-2007/112288
- WO-A-2007/131775
- WO-A-2007/144194
- WO-A-2008/139984
- US-A- 5 132 112
- US-A1- 2002 173 549
- US-A1- 2004 265 919
- US-A1- 2005 118 263
- QIU YI ET AL: "Design and synthesis of gentiohexaosyl derivatives for an ANP receptor antagonist, HS-142-1" BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, Bd. 60, Nr. 8, 1. Januar 1996 (1996-01-01), Seiten 1308-1316, XP008106173 ISSN: 0916-8451 [gefunden am 2008-02-08] in der Anmeldung erwähnt
- RITCHIE K ET AL: "The neuroprotective effects of caffeine - A prospective population study (the Three City Study)" NEUROLOGY, Bd. 69, Nr. 6, August 2007 (2007-08), Seiten 536-545, XP002538288 ISSN: 0028-3878
- ON BEHALF OF THE PARTICIPANTS OF THE INTERNATIONAL PSYCHOGERIATRIC ET AL: "Mild cognitive impairment" LANCET THE, LANCET LIMITED. LONDON, GB, Bd. 367, Nr. 9518, 15. April 2006 (2006-04-15), Seiten 1262-1270, XP025094106 ISSN: 0140-6736 [gefunden am 2006-04-15] in der Anmeldung erwähnt
- DALY J W: "Caffeine analogs: biomedical impact" CMLS CELLULAR AND MOLECULAR LIFE SCIENCES, BIRKHÄUSER-VERLAG, BA, Bd. 64, Nr. 16, 18. Mai 2007 (2007-05-18), Seiten 2153-2169, XP019536852 ISSN: 1420-9071
- MOGI ET AL: "Inhibition of cognitive decline in mice fed a high-salt and cholesterol diet by the angiotensin receptor blocker, olmesartan" NEUROPHARMACOLOGY, PERGAMON PRESS, OXFORD, GB, Bd. 53, Nr. 8, 22. November 2007 (2007-11-22), Seiten 899-905, XP022358182 ISSN: 0028-3908
- SUO ZHIMING ET AL: "Soluble Alzheimers beta-amyloid constricts the cerebral vasculature in vivo" NEUROSCIENCE LETTERS, Bd. 257, Nr. 2, 27. November 1998 (1998-11-27), Seiten 77-80, XP002538289 ISSN: 0304-3940
- GREENWOOD TIFFANY A ET AL: "Genome-wide linkage analysis of chromogranin B expression in the CEPH pedigrees: implications for exocytotic sympathochromaffin secretion in humans" PHYSIOLOGICAL GENOMICS, Bd. 18, Nr. 1, 17. Juni 2004 (2004-06-17), Seiten 119-127, XP002538290 ISSN: 1094-8341
- anonymous: "Current Alzheimer's Treatments", , pages 1-2, Retrieved from the Internet: URL:http://www.alz.org/research/science/al zheimers_disease_treatments.asp [retrieved on 2014-12-17]
- MELANDER O ET AL: "Plasma proneurotensin and incidence of diabetes, cardiovascular disease, breast cancer, and mortality", JAMA THE JOURNAL OF THE AMERICAN MEDICAL ASSOCIATION, AMERICAN MEDICAL ASSOCIATION, US, vol. 308, no. 14, 10 October 2012 (2012-10-10), pages 1469-1475, XP002699121, ISSN: 0098-7484, DOI: 10.1001/JAMA.2012.12998
- O. Melander ET AL: "Validation of Plasma Proneurotensin as a Novel Biomarker for the Prediction of Incident Breast Cancer", Cancer epidemiology, biomarkers & prevention, vol. 23, no. 8, 1 August 2014 (2014-08-01) , pages 1672-1676, XP055155246, ISSN: 1055-9965, DOI: 10.1158/1055-9965.EPI-13-1200
- anonymous: "ACE-Hemmer gegen AlzheimerBlutdrucksenker verzögern DemenzMedikamente gegen Bluthochdruck senken Alzheimer-Risiko", , 31 July 2013 (2013-07-31), page 1, Retrieved from the Internet: URL:http://www.lifeline.de/themenspecials/ alzheimer/blutdrucksenker-verzoegern-demen z-id111821.html [retrieved on 2014-12-19]
- S. J. Mandrekar ET AL: "Predictive biomarker validation in practice: lessons from real trials", Clinical Trials, vol. 7, no. 5, 14 April 2010 (2010-04-14), pages 567-573, XP055167114, ISSN: 1740-7745, DOI: 10.1177/1740774510368574
- "Oxford Centre for EvidenceBased Medicine 2011 Levels of Evidence", , 2011,
- D. L. SACKETT ET AL: "Rules of evidence and clinical recommendations on the use of antithrombotic agents", CHEST, vol. 95, no. 2, 1 February 1989 (1989-02-01), pages 2S-4S, XP055167119, ISSN: 0012-3692, DOI: 10.1378/chest.95.2.2S
- Pg Shekelle ET AL: "LEVELS OF EVIDENCE AND GRADES OF RECOMMENDATIONS RESEARCH QUICK TIPS FROM THE HEALTH SCIENCES LIBRARIES Bio-Medical Library 505 Essex Street A Directly based on Level I evidence B Directly based on Level II evidence or extrapolated recommendations from Level I evidence C Directly based on Le", West J Med, 1 June 1999 (1999-06-01), pages 348-51, XP055167124, Retrieved from the Internet: URL:http://hsl.lib.umn.edu/sites/default/f iles/Levels of Evidence.pdf [retrieved on 2015-02-04]
- M. S. Pepe ET AL: "Pivotal Evaluation of the Accuracy of a Biomarker Used for Classification or Prediction: Standards for Study Design", JNCI Journal of the National Cancer Institute, vol. 100, no. 20, 7 October 2008 (2008-10-07), pages 1432-1438, XP055167125, ISSN: 0027-8874, DOI: 10.1093/jnci/djn326
- Curriculum Vitae Dr. med. Volker Liebenberg
- Declaration of Dr. Volker Liebenberg to EP09705184
- BUYSE MARC ET AL: "Validation and clinical utility of a 70-gene prognostic signature for women with node-negative breast cancer", JOURNAL OF THE NATIONAL CANCER INSTITUTE, OXFORD UNIVERSITY PRESS, GB, vol. 98, no. 17, 6 September 2006 (2006-09-06), pages 1183-1192, XP002566232, ISSN: 0027-8874, DOI: 10.1093/JNCI/DJJ329

## Beschreibung

Die vorliegende Erfindung betrifft ein neues *in vitro* Verfahren zur Risikostratifizierung von Patienten mit leichten kognitiven Störungen zum Zweck der Ermittlung von therapiebedürftigen Patienten, die ein erhöhtes Risiko bezüglich einer Verschlechterung ihrer ursprünglich leichten kognitiven Störungen und bezüglich der Entwicklung einer klinisch manifesten neurodegenerativen Erkrankung vom Typ Alzheimer Demenz aufweisen, und zu dem Zweck, derartige therapiebedürftige Patienten einer Therapie mit bestimmten Mitteln zuzuführen, deren Eignung zu Therapiezwecken im vorliegenden Zusammenhang eine Erkenntnis ist, die einen wesentlichen Teil der vorliegenden Erfindung darstellt.

Ferner betrifft die Erfindung ein Verfahren zur präventiven Behandlung von aufgrund einer Biomarker-Messung ermittelten bestimmten Risiko-Patienten mit leichten kognitiven Störungen mit Herz-Kreislauf-Mitteln zur Verhinderung oder Verzögerung der Ausbildung von klinisch manifesten neurodegenerativen Störungen von Typ Alzheimer Demenz bzw. die Verwendung der Wirksubstanzen von Herz-Kreislauf-Mitteln und die Verwendung von ANP Rezeptor Antagonisten in einem solchen Verfahren sowie zur Herstellung von Arzneimitteln für den genannten Zweck der Verhinderung oder Verzögerung der Ausbildung von klinisch manifesten neurodegenerativen Störungen vom Typ Alzheimer Demenz.

Die Patienten, für die im Rahmen der vorliegenden Anmeldung eine retrospektive Abschätzung ihres Anfangsrisikos zur späteren Entwicklung einer nachgewiesenen klinisch manifesten neurodegenerativen Erkrankung erfolgte, und bei denen außerdem aus ihrer Vorgeschichte (Krankenakte) Faktoren ermittelt wurden, die sich retrospektiv als Prognose verbessernd erwiesen, sind Patienten mit der Diagnose "leichte kognitive Störungen" (abgekürzt: LKS). In der englischsprachigen Literatur werden die Kriterien für eine Feststellung "leichter kognitive Störungen" in jüngerer Zeit vor allem unter dem Begriff "mild cognitive impairment" (abgekürzt: MCI) abgehandelt. Es wird diesbezüglich beispielsweise verwiesen auf Serge Gauthier et al., "Mild cognitive impairment" in: Lancet 2006, 367:1262-1270, sowie ergänzend auf Ronald C. Petersen et al., "Mild Cognitive Impairment, Clinical Characterization and Outcome", In: Arch Neurol. 1999, 56:303-308, oder Ronald C. Petersen et al., "Current Concepts in Mild Cognitive Impairment" in: Arch Neurol. 2001; 58:1985-1992. Eines der im Zusammenhang mit der Feststellung eines LKS- oder MCI-Zustands eines Patienten angewandten Testverfahren ermittelt einen bestimmten, als "Mini Mental State" (MMS) bezeichneten Kennwert für einen bewerteten Patienten, vgl. Marshal F. Folstein et al., "MINI-MENTAL STATE - a practical method for grading the cognitive state of patients for The clinician", in: J. Psychiat. Res., 1975, 189-198.

Es ist bekannt, dass nur bei einem Teil derjenigen Patienten, bei denen zu einem bestimmten Zeitpunkt auf der Basis eigener Beobachtungen, oder aufgrund von Beobachtungen ihrer Umwelt, "leichte kognitive Störungen" (LKS) festgestellt werden bzw. die die Kriterien für "mild cognitive impairment" (MCI) erfüllen, in der Folge eine fortschreitende Verschlechterung ihres Zustand zu beobachten ist. Bestimmte Patienten entwickeln allmählich das volle klinische Symptombild einer neurodegenerativen Erkrankung, z.B. einer solchen vom Typ Alzheimer Demenz (AD). Bei anderen entwickelt sich eine Demenz anderen Typs, z.B. einer vaskuläre Demenz. Bei einem anderen Teil der LKS- bzw. MCI-Patienten tritt dagegen keine nennenswerte Verschlechterung auf, und manchmal verschwinden die ursprünglichen Symptome sogar wieder. Die erstgenannte Gruppe, bei der sich eine neurodegenerative Erkrankung entwickelt, wird auch als Gruppe der "Konverter nach Alzheimer" (converter) bezeichnet, während die letztgenannte Gruppe die Gruppe der "Nicht-Konverter" (non-converter) bildet.

Es wäre bei der beschriebenen Ausgangssituation wünschenswert, innerhalb der Gruppe der Patienten, für die eine Diagnose "leichte kognitive Störungen" gestellt wird, bereits bei der Diagnosestellung das individuelle Risiko, ob die einzelnen Patienten wahrscheinlich zur Gruppe der "Konverter nach Alzheimer" gehören oder nicht, abschätzen zu können, damit bei einem hohen festgestellten Risiko früh mit präventiven oder therapeutischen Maßnahmen begonnen werden kann (wenn derartige Maßnahmen überhaupt existieren), während bei Feststellung eines nur geringen Risikos einer solchen Konversion unnötige Maßnahmen vermieden werden könnten und die betroffenen Patienten psychisch erheblich entlastet würden.

Es wäre ferner wünschenswert, Patienten, für die ein erhöhtes Risiko zur Konversion nach Alzheimer besteht, einer Behandlung zuführen zu können, deren langfristige Wirksamkeit im Sinne einer Vermeidung oder Verzögerung einer Konversion gewährleisten ist.

Der vorliegenden Erfindung liegt die Aufgabe zugrunde, ein *in vitro* Verfahren zu schaffen, das eine Identifizierung der Gruppe der Konverter nach Alzheimer mit einer hohen statistischen Wahrscheinlichkeit ermöglicht, und das ferner eine Basis für die Entscheidung liefert, die ermittelten Konverter einer wirksamen präventiven Behandlung zuzuführen.

Diese Aufgaben werden durch Verfahren und deren bevorzugte Ausgestaltungen gelöst, wie sie der Fachmann den Patentansprüchen entnehmen kann.

Die Erfindung verkörpert sich in einem ihrer Aspekte in einem analytischen *in vitro* Verfahren, das in einer Gruppe von Patienten, für die aufgrund klinischer Befunde die Diagnose "leichte kognitive Störungen" gestellt wurde, bereits bei Diagnosestellung die Ermittlung der Patienten mit einem hohen Risiko einer späteren Konversion nach Alzheimer ermöglicht und das es ferner ermöglicht, für diese Patienten eine konkrete Therapieempfehlung auszusprechen.

Im Rahmen der vorliegenden Erfindung wird daher als eine erste Stufe ein analytisches *in vitro* Verfahren zur Ermittlung der Konzentrationen von kreislaufrelevanten Peptid-Biomarkern in Patientenproben (Plasma, Serum) angewandt, und bei Ermittlung von Konzentrationen, die im Vergleich mit entsprechenden Bezugskonzentrationen erhöht gefunden werden, wird den betreffenden Patienten eine Behandlung mit Herz-Kreislauf-Mitteln, insbesondere blutdrucksenkenden Mitteln oder eine Behandlung mit ANP Rezeptor Antagonisten empfohlen, da die nachfolgend beschriebenen Auswertungen ergaben, dass eine Behandlung mit blutdrucksenkenden Mitteln oder mit ANP Rezeptor Antagonisten das Risiko vermindert, dass sich eine festgestellte leichte kognitive Störung zu einer klinisch manifesten neurodegenerativen Erkrankung vom Typ Alzheimer Demenz (AD) verschlechtert.

Gegenstand der vorliegenden Erfindung ist somit ein *in vitro* Verfahren zur Risikostratifizierung von Patienten mit leichten kognitiven Störungen zum Zweck der Ermittlung therapiebedürftiger Patienten mit leichten kognitiven Störungen, umfassend:
- die Bestimmung mindestens eines kreislauf-relevanten Peptid-Biomarker in einer Probe einer biologischen Flüssigkeit aus dem Kreislauf eines Patienten mit der Diagnose einer leichten kognitiven Störung und
- die Zuordnung eines erhöhten Risikos für die Entwicklung einer klinisch manifesten neurodegenerativen Erkrankung zu einer erhöhten Konzentration für den mindestens einen kreislauf-relevanten Peptid-Biomarker, welche über einen biomarkerspezifischen Schwellenwert (Cut-Off) im Bereich üblicher Konzentrationswerte für diesen Biomarker bei leichten kognitiven Störungen liegt, wobei der kreislaufrelevante Biomarker ausgewählt ist aus den Fragmenten MR-pro-ANP und/oder MR-proADM und wobei der Schwellenwert für MR-ProANP 60 - 90 pmol/l und der Schwellenwert für MR-ProADM 0,5 - 0,7 nmol/l ist, und wobei Patienten mit dem festgestellten höheren Risiko als therapiebedürftig für die Behandlung mit einem Arzneimittel identifiziert werden, welches einen oder mehrere Wirkstoffe von Herz-Kreislauf-Mitteln umfasst, ausgewählt aus der Gruppe umfassend: ANP-Rezeptor-Antagonisten, Adenosin-Rezeptor-Antagonisten, ACE-Hemmer, Angiotensin-II-Rezeptor-Antagonisten, Betablocker und blutdrucksenkende Diuretika und deren Kombinationen, um die Ausbildung klinisch manifester neurodegenerativer Störungen zu verzögern oder zu verhindern oder den Zustand der leichten kognitiven Störung zu bessern oder zu halten.

Entsprechend dem erfindungsgemäßen *in vitro.* Verfahren können Patienten mit dem festgestellten höheren Risiko zur Entwicklung einer klinisch manifesten neurodegenerativen Erkrankung als therapiebedürftig für die Behandlung mit einem Arzneimittel identifiziert werden.

Für den Patienten mit dem festgestellten höheren Risiko kann dann eine Empfehlung für eine Behandlung mit Arzneimitteln gemäß den folgenden Ausführungen ausgesprochen werden

Gemäß dem erfindungsgemäßen *in vitro V*erfahren ist die Probe bevorzugt eine Serum- oder Plasmaprobe.

Das erfindungsgemäße Verfahren ist dadurch gekennzeichnet, dass eine Behandlung mit einem Herz-Kreislauf-Mittel aus der Gruppe der blutdrucksenkenden Mittel empfohen wird, das ausgewählt ist aus der Gruppe der Hemmer des "Angiotensin Converting Enzyms" (ACE-Hemmer), der Angiotension-II-Rezeptor-Antagonisten (der "Sartane"), der Betablocker und der zur Blutdrucksenkung eingesetzten Diuretika. Alternativ kann eine Behandlung mit einem ANP Rezeptor Antagonisten oder einem Adenosin Rezeptor Antagonisten empfohlen werden.

In einer bevorzugten Ausführungsform wird die Bestimmung mit einem immundiagnostischen Bestimmungsverfahren durchgeführt. Des Weiteren ist bevorzugt, dass man Fragmente von Prohormon-Vorläufern kreislaufrelevanter Peptide mit einer gegenüber den eigentlichen kreislaufrelevanten Peptiden verminderten oder nicht feststellbaren physiologischen Wirkung bestimmt.

Das Verfahren ist dadurch gekennzeichnet, dass man die Konzentration eines den mittleren Abschnitt des pro-ANP enthaltenden pro-ANP-Fragments (MR-pro-ANP) und/oder eines midregionalen pro-ADM-Fragments (MR-proADM), das die Aminosäuren 45-92 des Prä-Proadrenomedullins umfasst, bestimmt.

Gegenstand der vorliegenden Erfindung ist die Verwendung von& Peptid-Biomarkern, insbesondere MR-ANP und/oder MR-ADM zur Stratifizierung oder Ermittlung von Patienten mit leichten kognitiven Störungen, die ein erhöhtes Risiko zur Konversion in eine klinisch manifeste neurodegenerative Störung aufweisen.

Adenosin-Rezeptoren sind eine Klasse von purinergen, G-Protein gekoppelten Rezeptoren (GPCRs), deren endogener Ligand Adenosin ist. Man unterscheidet vier Subtypen: A1, A2A, A2B und A3, deren Verteilung im Körper und Funktion teilweise überlappend, und teilweise verschieden ist. Auch hinsichtlich der biochemischen Effekte und Siganlwege unterscheiden sich die Adenosin-Rezeptor Subtypen voneinander. Für jeden dieser Subtypen sind Agonisten und Antagonisten bekannt, einige Agonisten und Antagonisten, sind unspezifisch und zeigen Effekte mit allen Adenosin-Rezeptoren.

Antagonisten der ANP-Rezeptoren im Sinne der Erfindung sind therapeutisch verabreichbare Substanzen, die an ANP-Rezeptoren binden, dabei die Bindung des natürlichen Liganden ANP verhindern bzw. reduzieren und nicht zu einer Aktivierung der Ganylatcyclase mit der Folge der nicht- oder reduzierten Erhöhung des cGMP und in Folge dessen zu einer Reduzierung der Natriuretischen Wirkung des endogenen ANP führen.

Adenosin-Rezeptor-Antagonisten im Sinne der Erfindung sind therapeutisch verabreichbare Substanzen, die an Adenosin-Rezeptoren binden, dabei die Bindung des natürlichen Liganden Adenosin verhindern bzw. reduzieren und nicht zu einer Aktivierung der Ganylatcyclase mit der Folge der nicht- oder reduzierten Erhöhung des cGMP und in Folge dessen zu einer Reduzierung der Wirkung des endogenen Adenosins führen.

In einer besonders bevorzugten Ausführungsform der oben genannten Erfindung ist der ANP-Rezeptor-Antagonist ein Glucose-Capronsäure-Polymer (Glucose-Caproic Acid, insbesondere mit Capronsäure veresterte β1→ 6 Glucane). Ein besonders wichtiger Vertreter der ANP-Rezeptor-Antagonisten ist HS-142-1 (US 5,132,112). Sämtliche in US 5,132,112 erwähnten Antagonisten bilden Teil der hier vorliegenden Beschreibung und sind erfindungsgemäße ANP Rezeptor Antagonisten. Die HS-142-1-Verbindungen werden in US 5,132,112 wie folgt charakterisiert:
Diese Verbindungen umfassen lineares β1 → 6 Glucan, das mit Capronsäure verestert ist, wobei die Zahl der D-Glucosereste 28 und die der Capronsäurereste 11 beträgt.

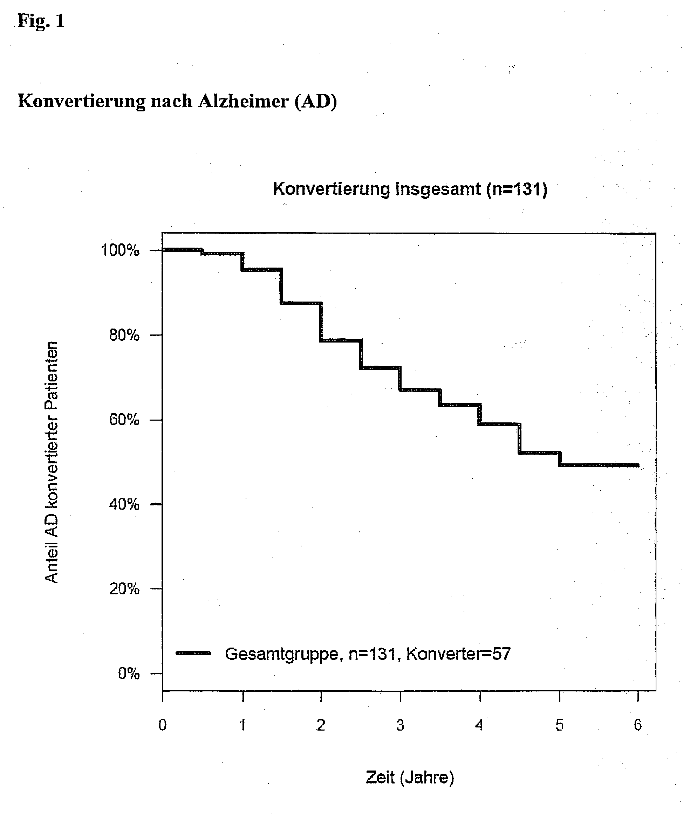

Diese Verbindungen haben im wesentlichen folgende physikochemischen Eigenschaften:
1) Beschaffenheit: weißes Pulver
2) Schmelzpunkt: 175-185 °C
3) Molekularformel: C₂₃₄H₃₉₂O₁₅₂
4) Massenspektrum (negatives FAB-Massenspektrum):
   Gefunden: M/Z 5536.5 (M-H)⁻
   Berechnet: M/Z 5637.3
5) Spezifische Rotation: [α]²⁴_{D} =-29.4° (c 0,24, wässrige Lösung)
6) Infrarotabsorptionsspektrum (KBr Tablettenmethode):
   cm-¹: 3420, 2930,1730,1635,1455,1380,1250,1170,1045
7) Ultraviolettes Absorptionsspektrum (wäßrige Lösung): Nur terminale Absorption sichtbar.
8) ¹H-NMR Spektrum (500 MHz, in D₂O): Wie in Abbildung 1 von US 5,132,112 gezeigt.
9) Farbreaktion:
   Positive Reaktionen mit Anisaldehyd, Schwefelsäure und Jodid.
   Negative Reaktionen mit Ninhydrin, Dinitrophenylhydrazin, Eisenchlorid,
   Bromkresolgrün und Dragendorff's Reagens.

Des weiteren ANP sind Rezeptor Antagonisten in WO 89/00428 erwähnt und sind ebenfalls Bestandteil dieser Beschreibung. Es ist auch bekannt, dass einige ANP Derivate antagonistische Eigenschaften aufweisen können (JP-A-225399/88) - auch diese Antagonisten sind Bestandteil dieser Beschreibung.

Lipo-Oligosaccharide, wie das HS-142-1 sind bekannt als Antagonisten des ANP Rezeptors, solche Antagonisten und deren Herstellung sind beschrieben in Yi Qiu, et al. Biosci. Biotech, Biochem, 60 (8) 1308-1315, 1996 und inkorporiert hiermit als solche sowie deren Herstellung. Insbesondere handelt es sich hier um Gentiohexaosylderivative als ANP Rezeptor Antagonisten. Beispiel für solche Derivate sind: HS-142-1, O-(3-O-caproyl-β-D-glucopyranosyl)-(1→6)-[O-(β-D-glucopyranosyl)- (1→6)-O-(3-O-caproyl-β-D-glucopyranosyl)-(1→6)]₂-D-glucopyranose (1), O-(3-O-hexyl-β-D-glucopyranosyl)-(1→6)-[O-(β-D-glucopyranosyl)- (1→6)-O-(3-O-hexyl-β-D-glucopyranosyl)-(1→6)]₂-D-glucopyranose (4) und O-(3-O-caproyl-2,4,6-tri-O-methyl-β-D-glucopyranosyl)- (1→6)]₂-2,3,4-tri-O-methyl-D-glucopyranose (2).

HS-142-1 ist ein ANP Antagonist und ist eine Mischung aus ß-1 -> 6 Oligoglukoside (EP:10-30) teils acyliert mit Caproylgruppen (5-15 in der Zahl).

In einer besonders bevorzugten Ausführungsform der oben genannten Erfindung ist der Adenosin-Rezeptor-Antagonist 1,3-dipropyl-8-(3-noradamantyl)xanthine.

Der ANP-Rezeptor-Antagonist wird verabreicht in einer Konzentration, die die Wirkung des natriuretischen Peptides insbesondere des ANP in folgender Weise erhöht Durch die erhöhte Bindung von Liganden (ohne entsprechende Aktivierung) an den ANP-Rezeptor wird der Rezeptor für seinen natürlichen Liganden blockiert. Erfolgt die Behandlung mit einer limitierten Konzentration an Antagonisten, (max 95 %, bevorzugt max. 90% der Rezeptoren werden gebunden, am meisten bevorzugt 80%) führt die durch besetzte Rezeptoren induzierte Neusynthese des Rezeptors zu einer insgesamt höheren Zahl an freien Rezeptoren als vor der Behandlung. Über die Erhöhung der Rezeptoren ergibt sich dann eine Hochregulierung der natriuretischen Wirkung des endogenen Hormone, da die Gleichgewichtslage zu freier Ligand/ gebundenem Ligand zugunsten des die biologische Wirkung erzeugenden gebundenen Liganden verschoben wird.

Dieser Effekt tritt ein, wenn die Rezeptoren mindestens zu 50 % durch Antagonisten belegt, bevorzugt zu 70%, am meisten bevorzugt 80 % aber nicht mehr als 95%. Dies entspricht üblicherweise im Falle von HS-142-1 einer Dosis von 0,01mg bis 0,1 mg HS 142-1/kg Körpergewicht, bevorzugt, 0,05 mg/ kg.

Dem Fachmann ist bekannt, dass die Konzentrationsbereiche für Antagonisten, wie auch ANP Rezeptor-Antagonisten, von der Affinität des Antagonisten abhängen. Ausgehend also von der ermittelten Belegung des Rezeptors und der Affinität des Rezeptor Antagonisten kann der Fachmann die jeweilige Dosis für den jeweiligen Antagonisten ermitteln. (Man kann sich bei dieser Ermittlung auch an der Plasmakonzentration von pro-ANP orientieren und beispielsweise bei > 74pmol/l (Plasmakonzentration) einen Bereich von 1x Pro-ANP - 100x Pro-ANP definieren, also bei 41 Plasma und einem Pro-ANP wert von 100pmol/l einen Bereich von 400pmol-40mmol Antagonisten / Verabreichung.

Der erwünschte Effekt des Adenosin-Rezeptor-Antagonisten tritt ein, wenn die Rezeptoren mindestens zu 50 % durch Antagonisten belegt, bevorzugt zu 70%, am meisten bevorzugt 80 % aber nicht mehr als 95%. Dies entspricht üblicherweise im Falle des bevorzugten 1,3-dipropyl-8-(3-noradamantyl)xanthine einer Dosis von 0,025 bis 1 mg/kg Körpergewicht, bevorzugt 0,5 mg/kg.

Insbesondere nützlich sind die vorgenannten Arzneimittel im Falle dass die klinisch manifeste neurodegenerative Störung vom Typ Alzheimer Demenz ist.

In einer bevorzugten Ausführungsform ist der Wirkstoff somit ein ANP-Rezeptor-Antagonist und wird in einer Dosis verabreicht, bei der mindestens 50%, bevorzugt 70%, am meisten bevorzugt 80 % aber nicht mehr als 95% der ANP-Rezeptoren belegt sind.

In einer anderen bevorzugten Ausführungsform ist der Wirkstoff ein Adenosin-Rezeptor-Antagonist und wird in einer Dosis verabreicht, bei der mindestens 50%, bevorzugt 70%, am meisten bevorzugt 80 %, aber nicht mehr als 95% der Adenosin-Rezeptoren belegt sind.

Weitere ANP-Rezeptor-Antagonisten können durch ein Screening-Verfahren identifiziert werden,umfassend die folgenden Schritte:
∘ Durchführung eines kompetitiven Rezeptorassays zur Ermittlung von Antagonistenkandidaten,
∘ Testen der Antagonistenkandidaten auf die Fähigkeit zur Blockade der der ANP-induzierten cGMP-Bildung, und
∘ Identifizieren der Antagonisten der Rezeptoren für das Atriale Natriuretische Peptid.

Potentielle ANP-Rezeptor-Antagonistenkandidaten können beispielsweise auf der Basis eines Radiorezeptorassays oder eines Radioimmunoassays erfolgen, in dem Kandidaten identifiziert werden, die radioaktiv markiertes ANP verdrängen. Solche Assays sind dem Fachmann bekannt und in der Literatur beschrieben, z.B. in Capper et. al (1990) Clin. Chem. 36/4, 656.

Potentielle Kandidaten werden dann auf ihre Fähigkeit zur Blockade der ANP-induzierten cGMP-Bildung getestet. Entsprechende Assays sind dem Fachmann bekannt und kommerziell erhältlich zum Beispiel von der Firma CisBio international (http://www.htrf.com/products/gpcr/cgmp/).

Im Zusammenhang mit der vorliegenden Erfindung gilt als ANP-Rezeptor-Antagonist eine Verbindung, die in dem im Folgenden beschriebenen Radiorezeptorassay einen IC₅₀-Wert kleiner als 10nmol/l vorzugsweise kleiner als 10pmol/l aufweist und in einem ANP-induzierten cGMP-Blockade Assay einen IC₅₀-Wert kleiner als 10nmol/l vorzugsweise kleiner 10pmol/l aufweist.

Der IC₅₀-Wert ist die Konzentration eines (potentiellen) Antagonisten (Inhibitors), bei der eine halbmaximale Verdrängung des Liganden (ANP) vom ANP-Rezeptors bzw. eine halbmaximale Blockierung der ANP-induzierten cGMP-Bildung beobachtet wird.

Der Radiorezeptorassay basiert auf der Verdrängung eines radiomarkierten ANP-Derivats von den Bindungsstellen des ANP-Rezeptors durch einen (potentiellen) Antagonisten und wird wie folgt durchgeführt:
1. Bereitstellung des rekonstituierten membrangebundenen ANP-Rezeptors (Guanylatcyclase A oder B) aus tierischem oder menschlichem Gewebe (bevorzugt Nierenzellpräparationen) oder geeignete Zelllinien mit natürlichem oder rekombinantem ANP-Rezeptor in einer Zellzahl von 100-100.000 Zellen/ Analyse in einer 50mM PBS-Pufferlösung, pH 7,5.
2. Zugabe von 1 fM bis 100 nM des (potentiellen) Antagonisten 2000-100.000 cpm radiomarkiertes ANP 1-28, radiojodiert am Tyr 28, ca. 2200 Ci/mmol (radiojodiert mittels der dem Fachmann bekannten Chloramine T-Methode) so dass das Verhältnis ANP/ANP-Rezeptor zwischen 100:1 und 1:100 beträgt.
3. Inkubation für 90 min bei Raumtemperatur.
4. Zentrifugation (30 min, 4°C, 2000 g) zur Trennung von Rezeptoren und überstand
5. Bestimmung der Konzentration von freiem (Überstand) und Rezeptor-gebundenem (Pellet) radiomarkierten ANP-Peptid durch Detektion der Radioaktivität mittels eines Gammazähler.
6. Aus der Konzentration Rezeptor-gebundenen radiomarkierten ANP-Peptids in Abhängigkeit von der Konzentration des zugegebenen Antagonisten wird der IC50-Wert ermittelt:

Der HTRF® (homogener zeitaufgelöste Fluoreszenz, Homogeneous Time Resolved Fluorescence) cGMP-Blockade Assay (CisBio international, Bagnols-sur-Cèze, Frankreich) basiert auf der Inhibierung der ANP-induzierten cGMP-Bildung durch einen (potentiellen) Antagonisten. Dabei macht man sich zu Nutze, dass die membrangebundene Guanylylcyclase (Type 1) durch ANP aktiviert wird. Bei dem cGMP-Blockade Assay wird in Gegenwart verschiedener Konzentrationen des (potentiellen) Antagonisten die Bildung von cGMP durch den vorliegenden ANP-Rezeptor bestimmt. In einem kompetitiven Immunassay wird anschließend die Menge des jeweils gebildeten cGMPs im Vergleich zur einem fluoreszenzmarkierten cGMP bekannter Konzentration mittels eines ebenfalls fluoreszenzmarkierten Anti-cGMP-Antikörpers bestimmt.

Der HTRF® cGMP-Assay wird wie folgt durchgeführt:
1. Bereitstellung, des membrangebundenen ANP-Rezeptors. (Guanylatcyclase) aus tierischem oder menschlichem Gewebe (bevorzugt Nierenzellpräparationen) oder geeignete Zelllinien mit natürlichem oder rekombinantem ANP-Rezeptor in einer Zellzahl von 100-100.000 Zellen/ Analyse. In 50mM PBS, 0,2% BSA, pH 7,2.
2. Zugabe von 1fM bis 100nM des (potentiellen) Antagonisten und 5-100.000 pM ANP 1-28, human.
3. Inkubation für 30 min bei 37 °C.
4. Bestimmung der gebildeten cGMP-Konzentration mittels Zugabe von cGMP-d2 in Lysis-Puffer und anti-cGMP-Cryptate (Anti-cGMP-Antikörper in Lysis-Puffer) in einem kompetetiven Immunassay. Die cGNT-Konzentration ist proportional zum Ratio der Fluoreszenzintensität bei 665/620 nm). Aus der Abhängigkeit des gebildeten cGMPs von der jeweiligen Antagonistenkonzentration wird der IC₅₀-Wert ermittelt.

Verfahren zur Diagnose (in einer weitgefassten Definition dieses Begriffs) von neurodegenerativen Erkrankungen durch Bestimmung von kreislauf-relevanten (vasoaktiven) Peptiden in z.B. Serum- oder Plasmaproben von einschlägigen Patienten werden beschrieben in älteren Anmeldungen der Anmelderin, und zwar insbesondere in DE 10 2006 023 175 A1 bzw. WO 2007/131 775 A1, wo eine statistisch relevante Korrelation zwischen der Schwere einer neurodegenerativen Erkrankung und den in den Patientenproben messbaren Konzentrationen von natriuretischen Peptiden nachgewiesen wird, sowie in DE 10 2006 027 818 A1 bzw. WO 2007/144 194 A1, wo beschrieben wird, dass eine noch bessere, statistisch relevantere Korrelation insbesondere dann erhalten wird, wenn man gleichzeitig mehr als ein vasoaktives Peptid berücksichtigt, und zwar insbesondere in Form einer Kombination der Konzentrationen von vasodilatorisch wirkenden Peptiden einerseits und vasokonstriktiv wirkenden Peptiden in der Patientenpröbe andererseits.

Die in den genannten älteren Anmeldungen beschriebenen Messungen erfolgten unter Verwendung von Proben (insbesondere Plasmaproben) von Patienten, für die bereits eine Diagnose "entwickelte neurodegenerative Erkrankung" vorlag, oder bei denen wenigstens der Verdacht bestand, dass sie an einer Frühform einer solchen Erkrankung litten.

Die Messungen wurden nicht für Prognosezwecke ausgewertet, da die weitere gesundheitliche Entwicklung der einzelnen Patienten, deren Proben vermessen worden waren, nicht über längere Zeiträume verfolgt wurde und zu den vorher gemessenen Biomarker-Konzentrationen in Beziehung gesetzt wurde.

Die genannten älteren Anmeldungen enthalten jedoch ausführliche Diskussionen der anerkannten klinischen Definitionen verschiedener Typen von neurodegenerativen Erkrankungen, und zur Problematik ihrer zutreffenden Diagnose. Da die vorliegende Anmeldung mindestens teilweise auf den in den beiden genannten Anmeldungen beschriebenen Untersuchungsbefunden aufbaut, wird der interessierte Fachmann zur Ergänzung der nachfolgenden Ausführungen ausdrücklich auf den gesamten Inhalt der beiden älteren Anmeldungen verwiesen.

Wie erläutert wird, sind neurodegenerative Erkrankungen (Demenzerkrankungen) generell sich eher langsam entwickelnde, chronische Erkrankungen von nichtinfektiöser Ätiologie. Die für eine zuverlässige Diagnosestellung erforderlichen langen Beobachtungszeiträume stellen sowohl im Hinblick auf die Diagnose und Prognose als solche, aber auch im Hinblick auf die Erforschung der genannten Erkrankungen und die Entwicklung wirksamer Präventions- und Therapiestrategien ein erhebliches Problem dar.

Generell werden als Demenzerkrankungen (Dementia) Krankheiten bezeichnet, für die ein gemeinsames Merkmal der Verlust erworbener intellektueller Fähigkeiten, vor allem des Gedächtnisses, und des normalen Persönlichkeitsniveaus als Folge von Hirnschädigungen ist. Treten Demenzerscheinungen vor dem hohen Alter bereits im mittleren Lebensalter auf, werden sie als präsenile Demenz-Erkrankungen bezeichnet.

Bei den Demenzerkrankungen unterscheidet man auf der Basis der für sie typischen Symptome und der postmortal feststellbaren hirnpathologischen Veränderungen verschiedene Erkrankungen bzw. Gruppen von Erkrankungen:
Von diesen ist die Alzheimer Demenz (AD) (Alzheimer Krankheit; Morbus Alzheimer) die häufigste neurodegenerative Demenz-Erkrankung, Sie macht 2/3 aller Demenzfälle aus und stellt auch das praktisch wichtigste Anwendungsgebiet für die vorliegende Erfindung dar. AD zeichnet sich durch drei wichtige, allerdings erst *post mortem* mit Sicherheit feststellbare pathologische Merkmale aus: die Bildung von Amyloid-Plaques und neurofibrillären Bündeln sowie den Verlust an Nervenzellen (vgl. ergänzend die Beschreibungseinleitung zu den o.g. älteren Anmeldungen).

Weitere Demenzerkrankungen, die im Rahmen der vorliegenden Anmeldung von geringerer Bedeutung sind und daher hier nicht im Detail diskutiert werden, sind die sogenannten vaskulären Demenzen (vascular dementia; VAD), bei denen eine Demenz aufgrund von Durchblutungsstörungen im Gehirn entsteht. Es gibt unterschiedliche Typen von VAD, wobei die Multi-Tnfarkt-Demenz (MID) und die subcorticale VAD (auch als Binswanger'sche Erkrankung bezeichnet) die häufigsten Formen darstellen. Ferner sind der Vollständigkeit halber zu zu nennen die Demenz mit Lewy-Körperchen (dementia with lewy-bodies: DLB) und die Frontotemporale Demenz (FTD), auch als Pick'sche Krankheit bezeichnet.

Die für die verschiedenen Demenzerkrankungen typischen Gehirnveränderungen können an lebenden Patienten naturgemäß nicht direkt festgestellt werden, und apparatemedizinische Untersuchungen der Gehirnfunktionen mittels z.B. Röntgen- oder Kernspin-Tomographie sind aufwändig und teuer. Demenzerkrankungen sind daher auch heute noch unterdiagnostiziert oder unrichtig diagnostiziert, und entsprechend mangelhaft sind die Prognosemöglichkeiten und Therapiemöglichkeiten. Die Arbeiten der Anmelderin zielen darauf ab, die Diagnose- und Prognosemöglichkeiten von neurodegenerativen Erkrankungen, insbesondere die Diagnose von AD, durch Entwicklung von relativ einfach einsetzbaren Messverfahren zu verbessern, im Rahmen derer in Proben aus dem Kreislauf der Patienten gut messbare Biomarker bestimmt werden.

Zu den vielversprechenden erfindungsgemäßen Biomarkern gehören verschiedene kreislaufrelevante Peptid-Biomarker, von denen man im Rahmen des Verfahrens der vorliegenden Erfindung vorzugsweise Biomarker in Form vasodilatorisch wirkender Peptide bestimmt, und zwar insbesondere natriuretische Peptide wie ANP, BNP und/oder CNP und/oder das Peptid Adrenomedullin (ADM).

Die Bestimmung der Konzentrationen von natriumdiuretischen Peptiden wird exemplarisch an der Bestimmung der Konzentration von ANP gezeigt. ANP wird vorzugsweise als Konzentration eines proANP-Fragments mit Hilfe eines Assays der Anmelderin bestimmt, der einen mittleren Abchnitt des proANP (MR-proANP) erkennt. Ein solcher Assay ist beschrieben in EP 1 562 984 B1 bzw. in: Nils G. Morgenthaler et al., Immunoluminometric Assay for the Midregion of Pro-Atrial Natriuretic Peptide in Human Plasma, Clinical Chemistry 50:1, 2004, 234-236.

Die Konzentration von Adrenomedullin (ADM) wird vorzugsweise als Konzentration eines midregionalen proADM-Fragments (MR-proADM) bestimmt, das die Aminosäuren 45-92 des Prä-Proadrenomedullins umfasst. Ein geeigneter Assay ist beschrieben in EP 1 488 209 B1 bzw. WO 2004/090546 bzw. in Nils G. Morgenthaler et al., Measurement of Midregional Proadrenomedullin in Plasma with an Immunoluminometric Assay, Clinical Chemistry 51:10, 2005, 1823-18.29.

Wenn in dieser Anmeldung ein Begriff wie "Konzentration eines Peptid-Biomarkers" oder ein ähnlicher Begriff verwendet wird, ist daher nicht, im Sinne einer einengenden Gleichsetzung, nur die in der biologischen Probe messbare stationäre Konzentration des eigentlichen vasoaktiven Peptids gemeint.

Die wichtigsten im Rahmen der vorliegenden Erfindung diskutierten pathophysiologisch freigesetzten eigentlichen vasoaktiven Peptide liegen nur zu einem geringeren Teil frei bzw. unbehindert messbar in biologischen Flüssigkeiten vor. Wesentliche Teile der pathophysiologisch freigesetzten eigentlichen vasoaktiven Peptide werden der biologischen Flüssigkeit durch Bindung an Rezeptoren und andere Membran- oder Gefäßstrukturen rasch entzogen und/oder abgebaut.

Die Messung von inaktiven, aus den gleichen Vorläufer-Propeptiden gebildeten Co-Peptiden, wie sie im Rahmen der vorliegenden Erfindung unter Verwendung der bereits genannten Assays der Anmelderin bevorzugt erfolgt, spiegelt, anders als die messbare momentane Konzentration in einer biologischen Flüssigkeit, die Freisetzung der vasoaktiven Peptide im Sinne von "Wirkkonzentrationen" über einen längeren Zeitabschnitt wieder und gestattet eine indirekte Miterfassung auch gebundener oder schnell abgebauter Anteile des ursprünglich freigesetzten vasoaktiven Peptids. Das führt in Verbindung mit der höheren Stabilität solcher Co-Peptide zu höheren messbaren Absolutkonzentrationswerten für den zu interessierenden Analyten in der biologischen Flüssigkeit, z.B. in Serum oder Plasma.

Bei Erkrankungen mit einem eher chronischen Verlauf ohne plötzliche Verschlechterungen oder Verbesserungen des Zustands des Patienten, wie es bei Demenzerkrankungen die Regel ist, besteht allerdings eine erhebliche Wahrscheinlichkeit dafür, dass sich bezüglich der verschiedenen krankheitsrelevanten Analyten ein nur wenig variabler, sich nur langsam verändernder stationärer Gesamtzustand ausbildet. Die stationär in der biologischen Flüssigkeit des Patienten messbaren Konzentrationen eines aktiven Analyten vom Typ eines Peptid-Biomarkers, im vorliegenden Falle eines vasoaktiven Peptids, dürften daher im wesentlichen proportional zu den über längere Zeiträume pathophysiologisch freigesetzten Mengen des gleichen Analyten sein, wie sie in Form des physiologisch inaktiven Co-Peptids gemessen werden können. Das bedeutet, dass sich die bei der bevorzugten Bestimmung inaktiver Co-Peptide gemäß der vorliegenden Erfindung feststellbaren Abweichungen von den Kontrollwerten Gesunder, sowie der krankheitstypische Gang dieser Abweichungen, auch in den in der Regel niedrigeren stationären Konzentrationen der aktiven Analyten widerspiegeln sollten, so dass der konkreten Wahl der gemessenen "Analytenkonzentrationen" kein entscheidender qualitativer Einfluss auf die diagnostische Auswertung zukommen sollte.

Im Rahmen der vorliegenden Erfindung konnte für Messzwecke auf eine Sammlung von Proben von Patientenplasmen zurück gegriffen werden, die Plasmaproben-verschiedener individueller Patienten umfasste, die zum Zeitpunkt der Diagnosestellung "leichte kognitive Störungen" (t=0) gewonnen worden waren ("LKS-Patienten"). Die anschließende Entwicklung des Gesundheitszustands der einzelnen Patienten war über einen Zeitraum von 6 Jahre dokumentiert.

Es wurde gefunden, dass die Konzentrationen der untersuchten Biomarker in Plasma von LKS-Patienten gegenüber Kontrollpersonen ohne kognitive Störungen im Mittel erhöht waren, und dass innerhalb des Bereichs der gefundenen Konzentrationen ein Cut-Off-Konzentrationswert definiert werden konnte, der eine Unterscheidung von LKS-Patienten mit einem geringen Risiko zur späteren Konversion von LKS-Patienten mit einem erheblichen Risiko zur späteren Konversion nach AD erlaubt. Die Unterscheidung der Patientengruppen wurde bei gleichzeitiger Betrachtung von zwei Biomarkern noch verbessert.

Indem der Erfinder, wie im experimentellen Teil gezeigt werden wird, darüber hinaus die Messergebnisse gestützt auf die dokumentierten Krankengeschichten noch zusätzlich daraufhin untersuchte, ob Patienten, für die keine oder nur eine deutlich verzögerte spätere Konversion im Sinne der Entwicklung einer klinisch manifesten neurodegenerativen Erkrankung (Alzheimer Demenz) beobachtet wurde, bereits aus anderen Gründen einer Behandlung mit bestimmten Medikamenten oder Medikamententypen unterzogen worden waren, konnte er feststellen, dass sich offensichtlich eine Behandlung mit Herz-Kreislauf-Mitteln, insbesondere blutdrucksenkenden Mitteln, positiv auf die Prognose im Sinne einer Vermeidung oder Verzögerung der "Konversion nach AD" ausgewirkt hatte.

Daraus ergab sich, dass für Patienten mit leichten kognitiven Störungen bereits bei Diagnosestellung nicht nur eine Risikostratifizierung möglich ist, sondern dass man den Patienten mit einem ermittelten erhöhten Konversionsrisiko nach AD auch eine sinnvolle, derzeit nicht ins Behandlungsrepertoir der einschlägigen Fachärzte gehörende neue Behandlung empfehlen kann, nämlich eine Behandlung mit Herz-Kreislauf-Mitteln, insbesondere blutdrucksenkenden Mitteln, oder eine Behandlung mit ANP Rezeptor Antagonisten oder Adenosin-Rezeptor-Antagonisten.

Herz-Kreislauf Mittel bzw. blutdrucksenkende Mittel im obigen Sinne sind an sich bekannte Mitteln, die klinisch bereits umfangreich getestet wurden. Die heute therapeutisch bzw. präventiv routinemäßig eingesetzten blutdrucksenkenden Mittel gehören im Wesentlichen zu vier unterschiedlichen Medikamententypen, nämlich insbesondere zu Medikamenten aus der Gruppe der Hemmer des "Angiotensin Converting Enzyms" (ACE-Hemmer), der Angiotension-II-Rezeptor-Antagonisten (der "Sartane"), der Betablocker und der zur Blutdrucksenkung eingesetzten Diuretika.

Nähere Einzelheiten zu den heute eingesetzten Typen dieser Medikamente bzw. den einzelnen der in großem Umfang verschriebenen Vertreter der genannten Wirkstoffklassen lassen sich der sehr umfangreichen Fachliteratur entnehmen, die sich mit Maßnahmen zur Senkung des Blutdrucks von Patienten mit erhöhtem Blutdruck befasst.

Lediglich beispielhaft werden genannt als Vertreter der ACE-Hemmer die Arzneistoffe Captopril, Enalapril, Lisinopril und Ramipril, als Vertreter der Angiotension-II-Rezeptor-Sutityp-1-Antagonisten (AT1-Antagonisten; Sartane) die Arzneistoffe Losartan, Valsartan, Candesartan, Irbesartan, Telmisartan, Eprosartan und Olmesartan, und als Diuretika insbesondere die sogenannten Thiazid-Diuretika wie Hydrochlorothiazid und die Thiazid-Analoga wie Clorthalidon, Clopramid, Indepramid, Metolazon, Xipramid und Mefrusid.

Die nachfolgende Tabelle 1 fasst die Grundinformationen zu den verschiedenen Medikamententypen und ihren Wirkmechanismen kurz zusammen:

**Tabelle 1:**

| **Medikament** | **Wirkung** |
|---|---|
| | Sie blockieren die Wirkung des körpereigenen Proteins ACE, das für die Herstellung des Hormons Angiotensin-II (AT-II) verantwortlich ist. AT-II verengt die Blutgefäße und hält den Blutdruck hoch. Durch die Verringerung der ATII-Konzentration im Körper bleiben die Blutgefäße auf Dauer erweitert und der Blutdruck sinkt. |
| **ACE**-**Hemmer** | Das Herz muss gegen einen geringeren Widerstand arbeiten und wird entlastet. Zudem können sie einen krankhaften Umbau des Herzmuskels verlangsamen. ACE-Hemmer verbessern die Prognose der Herzinsuffizienz und sind gut verträglich. Häufig tritt Hustenreiz auf, der aber harmlos ist. |
| **Angiotensin**-**II-Rezeptor-Antagonisten** | Sie werden auch als "Sartane" bezeichnet und kompetieren mit Angiotensin-II um die Bindungsstellen des zugehörigen Rezeptors. Sie werden von verschiedenen Patienten besser vertragen bzw. zeigen bei verschiedenen Patienten eine bessere Wirkung als ACE-Hemmer und werden eingesetzt, wenn ein Patient keine ACE-Hemmer verträgt oder diese bei him nicht die gewünschte Wirkung zeigen. |
| **Betablocker** | Sie reduzieren die Wirkung der Stresshormone (Katecholamine) am Herzen. Stresshormone werden bei Herzinsuffizienz vermehrt ausgeschüttet und können zu Herzrhythmusstörungen führen. Betablocker verbessern die Prognose, da sie lebensbedrohlichen Herzrhythmusstörungen vorbeugen. |
| **Diuretika** | Spezielle harntrerberide Medikamente (Aldosteron-Antagonisten) verbessern die Prognose bei fortgeschrittenem Stadium. Die genaue Wirkungsweise ist bisher unklar. |

Vorzugsweise ist der ANP-Rezeptor-Antagonist ein Glucose-Caproic Acid Polymer.

Vorzugsweise ist der Adenosin-Rezeptor-Antagonist 1,3-Dipropyl-8-(3-Noradamantyl)Xanthin ("Rolofylline").

Der Begriff "Risikostratifizierung" (auch "Risikostratifikation" oder einfach "Stratifikation") bezeichnet in der Medizin das Abschätzen des Risikos, dass bei einem Patienten, oder bei einer Untergruppe eines Prüfkollektivs von Patienten, eine Erkrankung fortschreitet und zu zunehmend ernsteren Komplikationen oder zu einem vorgegebenen Endpunkt (z.B. Tod aufgrund der Erkrankung) führt. Zum Zweck einer Risikostratifizierung werden Risikofaktoren oder diagnostisch relevante Parameter erfasst, von denen bekannt ist, dass sie im Zusammenhang mit dem Fortschreiten einer Erkrankung und/oder mit dem Auftreten von Komplikationen stehen. Mit Hilfe verschiedener statistischer biometrischer Auswertungstechniken in Form von Tabellen, Algorithmen oder damit arbeitenden Computerprogrammen wird auf der Basis der erfassten Daten das individuelle Risiko eines Patienten oder das Risiko einer Patienten-Untergruppe ermittelt.

Man kann die Risikostratifizierungen auch als Instrument der Prognose betrachten, mit dem eine Prognose, zu verstehen als eine bestimmte Wahrscheinlichkeit, aus Prüfkriterien abgeleitet werden soll, die sich in vorausgehenden Untersuchungen als für die jeweilige Fragestellung relevant bzw. valide erwiesen haben, z.B. um rechtzeitig erforderliche Gegenmaßnahmen einzuleiten, wenn es für die sich entwickelnden Gesundheitsstörungen sinnvolle Präventionstherapien gibt, oder um zu vermeiden, dass auch solche Patienten belastenden und/oder teuren Therapie- und Präventionsmaßnahmen unterzogen werden, die derartige Maßnahmen mit überwiegender Wahrscheinlichkeit überhaupt nicht benötigen.

Ein bekanntes, an spezielle Testbedingungen angepasstes mathematisches Verfahren zur Risikostratifizierung ist die sog. Kaplan-Meier-Methode (auch "Kaplan-Meier-Schätzer" oder "Kaplan-Meier-Estimator"), die z.B. zur Überlebenszeitanalyse dann zur Anwendung kommt, wenn nicht alle im Test berücksichtigten Testpersonen für die gesamte Testdauer zu Verfügung stehen, z.B. weil sie erst später zu dem Patientenkollektiv hinzukommen, aus diesem ausscheiden, z.B. durch Tod, der nicht auf das untersuchte Risiko zurückgeht, oder weil sie innerhalb der Testdauer keine eindeutigen Befunde zeigen.

Das genannte Auswertverfahren, das genauer z.B. in Lehrbüchern der medizinischen Biometrie beschrieben wird und/oder zu dem sich unter den genannten Bezeichnungen im Internet Erläuterungen finden und für dessen Anwendung kommerzielle Computcrprogramme angeboten werden, wird im Rahmen der Auswertung der im nachfolgenden experimentellen Teil beschriebenen Messung von kreislauf-relevanten Peptid-Biomarkern im Plasma von solchen Patienten angewandt, für die eine Erstdiagnose "leichte kognitive Störungen" gestellt wurde. Bei Anwendung dieses Verfahrens werden sog. "Kaplan-Meier-Plots" erhalten, wie sie in den meisten Figuren der vorliegenden Anmeldung gezeigt werden.

Nachfolgend werden einige Ausführungsformen aufgezählt, welche nicht Bestandteil des Schutzumfangs der vorliegenden Anmeldung sind, aber zu deren Verständnis beitragen sollen:
1. Arzneimitteln, welches einen oder mehrere Wirkstoffe von Herz-Kreislauf-Mitteln umfasst, ausgewählt aus der Gruppe umfassend: ANP-Rezeptor-Antagonisten, Adenosin-Rezeptor-Antagonisten, ACE-Hemmer, Angiotensin-II-Rezeptor-Antagonisten, Betablocker und blutdrucksenkende Diuretika und deren Kombinationen zur Behandlung von Patienten mit leichten kognitiven Störungen um die Ausbildung klinisch manifester neurodegenerativer Störungen zu verzögern oder zu verhindern oder den Zustand der leichten kognitiven Störung zu bessern oder zu halten.
2. Arzneimittel gemäß Detail 1, wobei der ANP-Rezeptor-Antagonist ein Glucose-Capronsäure-Polymer ist.
3. Arzneimittel gemäß Detail 1, wobei der Adenosin-Rezeptor-Antagonist 1,3-Dipropyl-8-(3-Noradamantyl)Xanthin ist.
4. Arzneimittel gemäß einem der Details 1 oder 2, wobei der Wirkstoff ein ANP-Rezeptor-Antagonist ist und der Antagonist verabreicht wird in einer Dosis, bei der mindestens 50% der ANP-Rezeptoren belegt sind.
5. Arzneimittel gemäß einem der Details 1 oder 3, wobei der Wirkstoff ein Adensosin-Rezeptor-Antagonist ist und der Antagonist verabreicht wird in einer Dosis, bei der mindestens 50% der Adenosin-Rezeptoren belegt sind.
6. Arzneimittel gemäß einem der Details 1 bis 5, wobei die klinisch manifeste neurodegenerativer Störung vom Typ Alzheimer Demenz ist.
7. Arzneimittel gemäß einem der Details 1 bis 6, wobei zur Behandlung eine Patientengruppe ausgewählt wird, die aufgrund einer in ihrem Kreislauf messbaren Erhöhung von Kreislauf-relevanten Peptid-Biomarkern als Patienten mit einem erhöhten Risiko zur Ausbildung einer klinisch manifesten Alzheimer Demenz identifizierbar sind.
8. In vitro Verfahren zur Ermittlung therapiebedürftiger Patienten mit leichten kognitiven Störungen, bei dem man
   - in einer Probe einer biologischen Flüssigkeit aus dem Kreislauf eines Patienten mit der Diagnose einer leichten kognitiven Störung mindestens einen kreislaufrelevanten Peptid-Biomarker bestimmt und
   - einer Konzentration für den mindestens einen kreislauf-relevanten Peptid-Biomarker, die über einem biomarkerspezifischen Schwellenwert (Cut-Off) im Bereich üblicher Konzentrationswerte für diesen Biomarker bei leichten kognitiven Störungen liegt, ein erhöhtes Risiko zur Entwicklung einer klinisch manifesten neurodegenerativen Erkrankung zuordnet.
9. In vitro Verfahren gemäß Detail 8, wobei für den Patienten mit dem festgestellten höheren Risiko eine Empfehlung für eine Behandlung mit Arzneimitteln gemäß den Ansprüchen 1 bis 6 ausgesprochen wird.
10. Verfahren nach Detail 8 oder 9, dadurch gekennzeichnet, dass die Probe eine Serum-oder Plasmaprobe ist.
11. Verfahren nach einem der Details 8 bis 10, dadurch gekennzeichnet, dass die kreislaufrelevanten Peptid-Biomarker ausgewählt sind aus den natriuretisehen Peptiden ANP und/oder BNP und/oder Adrenomedullin (ADM) oder aus Fragmenten der jeweiligen zugehörigen Prohormone pro-ANP, pro-BNP oder pro-ADM.
12. Verfahren nach einem der Details 8 bis 11, dadurch gekennzeichnet, dass eine Behandlung mit einem Herz-Kreislauf Mittel aus der Gruppe der blutdrucksenkenden Mittel empfohlen wird, das ausgewählt ist aus der Gruppe der Hemmer des "Angiotensin Converting Enzyms" (ACE-Hemmer.) der Angiotensin-II-Rezeptor-Antagonisten (der "Sartane"), der Betablocker und der zur Blutdrucksenkung eingesetzten Diuretika.
13. Verfahren nach Detail 11, dadurch gekennzeichnet, dass man die Bestimmung mit einem immundiagnostischen Bestimmungsverfahren durchführt und dass man Fragmente von Prohormon-Vorläufern kreislaufrelevanter Peptide mit einer gegenüber den eigentlichen kreislaufrelevanten Peptiden verminderten oder nicht feststellbaren physiologischen Wirkung bestimmt.
14. Verfahren nach Detail 13, dadurch gekennzeichnet, dass man die Konzentration eines den mittleren Abschnitt des pro-ANP enthaltenden pro-ANP-Fragments (MR-pro-ANP) und/oder eines midregionalen pro-ADM-Fragments (MR-proADM), das die Aminosäuren 45-92 des Prä-Proadrenomedullins umfasst, bestimmt.
15. Screeningverfahren zum Identifizieren von Antagonisten der Rezeptoren für das Atriale Nätriuretische Peptid umfassend die Schritte:
   o Durchführung eines kompetitiven Rezeptorassays zur Ermittlung von Antagonistenkandidaten,
   ∘ Testen der Antagonistenkandidaten auf die Fähigkeit zur Blockade der der ANP-induzierten cGMP-Bildung, und
   ∘ Identifizieren der Antagonisten der Rezeptoren für das Atriale Natriuretische Peptid.
16. Verwendung von Kreislauf-relevanten Peptid-Biomarkern, insbesondere natriuretische Peptide ANP, BNP und/oder Adrenomedullin (ADM) oder aus Fragmenten davon oder Fragmenten der zugehörigen Prohormone pro-ANP, pro-BNP oder pro-ADM zur Stratifizierung oder Ermittlung von Patienten mit leichten kognitiven Störungen, die ein erhöhtes Risiko zur Konversion in eine klinisch manifeste neurodegenerative Störung aufweisen.
17. ANP-Rezeptor-Antagonist zur Verwendung als Medikament.
18. ANP-Rezeptor-Antagonist nach Detail 17, wobei der ANP-Rezeptor-Antagonist ein Glucose-Capronsäure-Polymer ist.

Nachfolgend im experimentellen Teil werden die Messungen, die der vorliegenden Erfindung zugrunde liegen, und die zugehörigen Auswertungen der Messergebnisse unter Bezugnahme auf mehrere Figuren und Tabellen noch näher erläutert.

Dabei wird auf zehn Figuren (Diagramme) Bezug genommen, deren Inhalt nachfolgend erläutert wird:
- Figur 1: zeigt einen sogenannten Kaplan-Meier-Plot, in dem die Entwicklung des Gesundheitszustands, im Sinne einer Konversion nach Alzheimer, d.h. einer Entwicklung einer AD, des Messkollektivs von 131 Patienten mit der ursprünglichen Diagnosestellung "leichte kognitive Störungen" auf der Basis der rein klinischen Diagnose über einen Zeitraum von mehr als sechs Jahren dargestellt ist. Im überwachten Zeitraum entwickelten 57 der 131 Patienten eine klinisch manifeste Alzheimer-Demenz (AD).

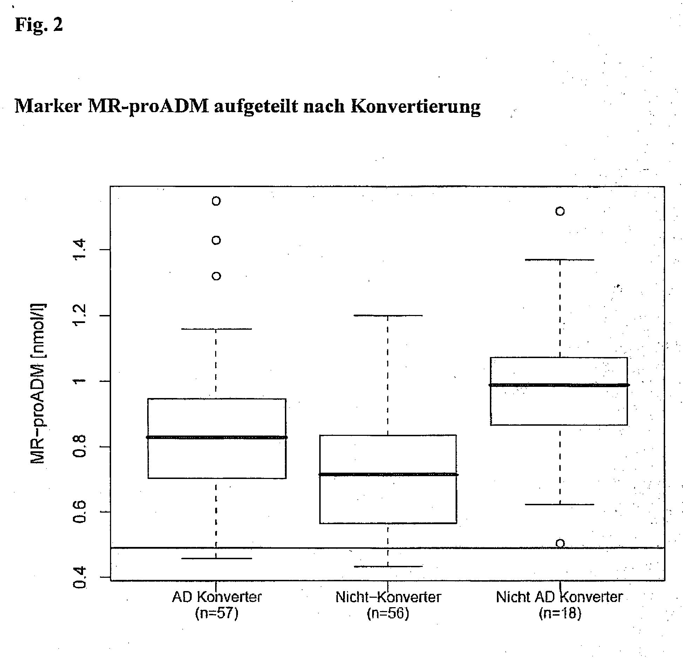

- Figur 2: zeigt die in den Plasmaproben der Patientengruppe aus Figur 1 gemessenen Konzentrationen für den Peptid-Biomarker MR-proADM bei Diagnosestellung (t=0), getrennt für die späteren Konverter nach Alzheimer (AD "Konverter), die Nicht-Konverter und Konverter nach anderen "Nicht-AD" Demenzformen.

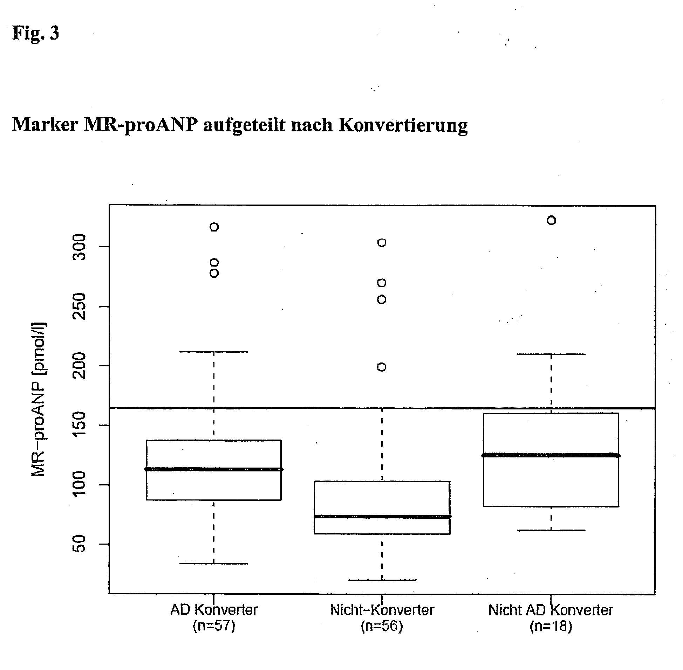

- Figur 3: zeigt die in den Plasmaproben der Patientengruppe aus Figur 1 gemessenen Konzentrationen für den Peptid-Biomarker MR-proANP bei Diagnosestellung (t=0), getrennt für die späteren Konverter nach Alzheimer (AD Konverter), die Nicht-Konverter und Konverter nach anderen "Nicht-AD" Demenzformen.

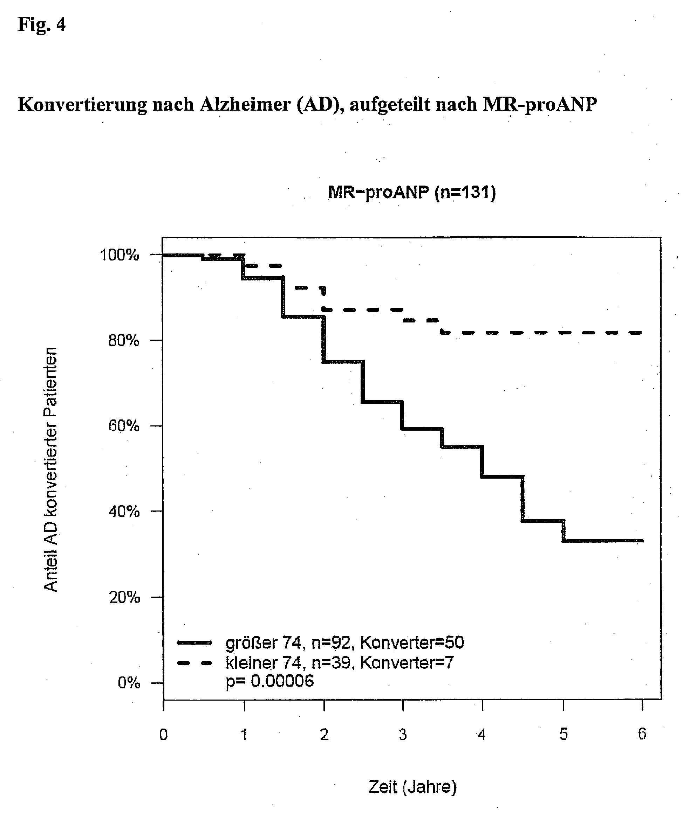

- Figur 4: zeigt einen Kaplan-Meier-Plot für die Konvertierung nach AD für zwei Gruppen von Patienten einmal mit einer gemessenen MR-proANP Konzentration, die über dem Cut-Off-Wert von 74 pmol/l (größer 74) lag, und zum anderen mit einer unter dem genannten Cut-Off Wert liegenden Konzentration (kleiner 74).

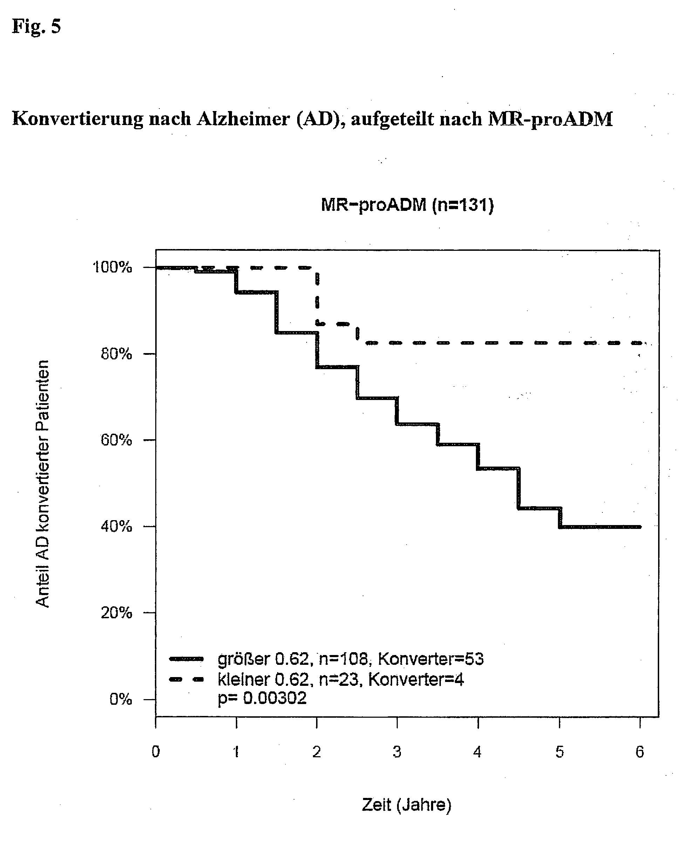

- Figur 5: zeigt einen Kaplan-Meier-Plot für die Konvertierung nach AD für zwei Gruppen von Patienten einmal mit einer gemessenen MR-proADM Konzentration, die über dem Cut-Off-Wert von mehr als 0,62 nmol/l(größer 0,62) lag, und zum anderen mit einer unter dem genannten Cut-Off-Wert liegenden Konzentration (kleiner 0,62).

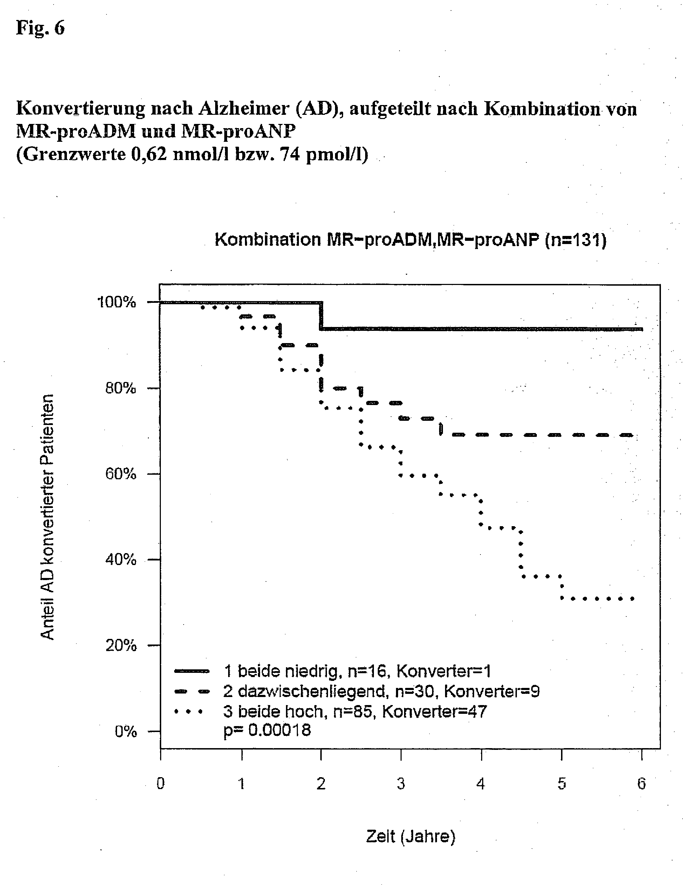

- Figur 6: zeigt einen Kaplan-Meier-Plot für die Konvertierung nach AD von Patienten, wobei die Plasmakonzentrationen für beide Peptid-Biomarker MR-proANP und pro-ADM gleichzeitig berücksichtigt wurden, und zwar unterschieden nach Patientengruppen, bei denen (a) die Konzentrationen für beide Biomarker unter den in den Figuren 4 und 5 angegebenen Cut-Off-Werten lagen (beide niedrig), (b) nur eine der Konzentrationen unter einem der in den Figuren 4 und 5 angegebenen Cut-Off-Wert lag (dazwischenliegend), und (c) die Konzentrationen für beide Biomarker über den in den Figuren 4 und 5 angegebenen Cut-Off-Werten lagen (beide hoch).

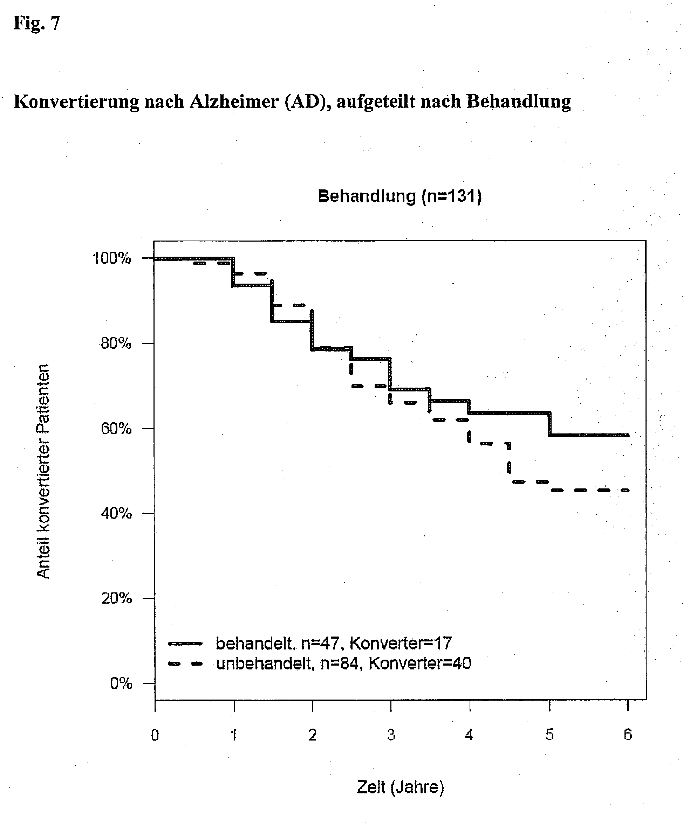

- Figur 7: zeigt die klinisch diagnostizierte Konvertierung nach AD der 131 LKS-Pätienten getrennt einerseits nach Patienten, die laut ihrer Krankenakte vor Diagnosestellung mit Herz-Kreislauf-Mitteln behandelt worden waren (behandeln), und andererseits nach Patienten, für die keine derartige Vorbehandlung dokumentiert war. Es ist auf der Basis einer solchen Auswertung kein signifikanter Unterschied zwischen den genannten Patientengruppen feststellbar.

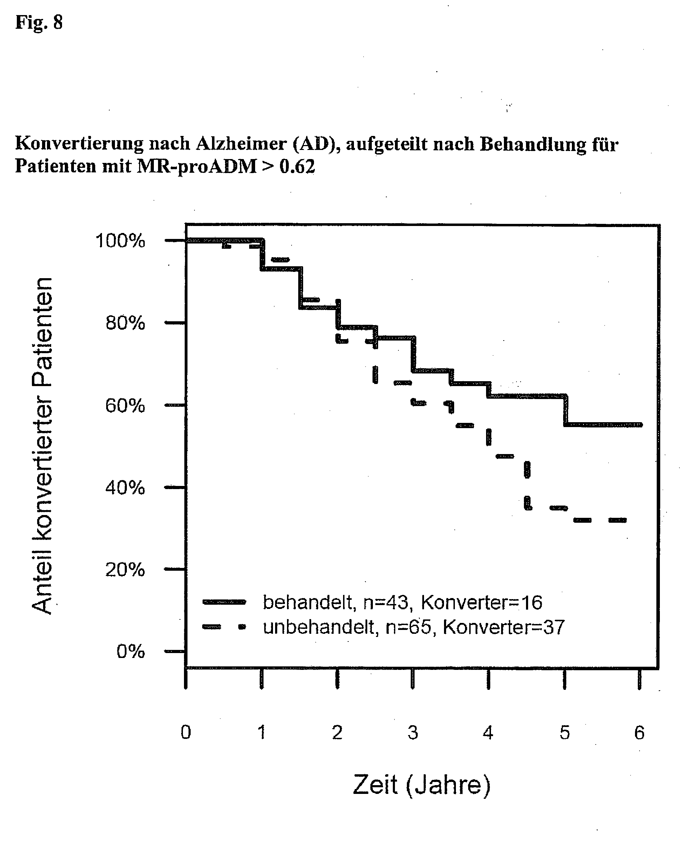

- Figur 8: zeigt die klinisch diagnostizierte Konvertierung nach AD einer Untergruppe von 108 Patienten von den 131 ursprünglichen LKS-Patienten, für die ein MR-proADM-Wert über dem Cut-Off von 0,62 nmol/l gemessen worden war, getrennt einerseits nach Patienten, die laut ihrer Krankenakte vor Diagnosestellung mit Herz-Kreislauf-Mitteln behandelt worden waren (behandelt), und andererseits nach Patienten, für die keine derartige Vorbehandlung dokumentiert war (unbehandelt).

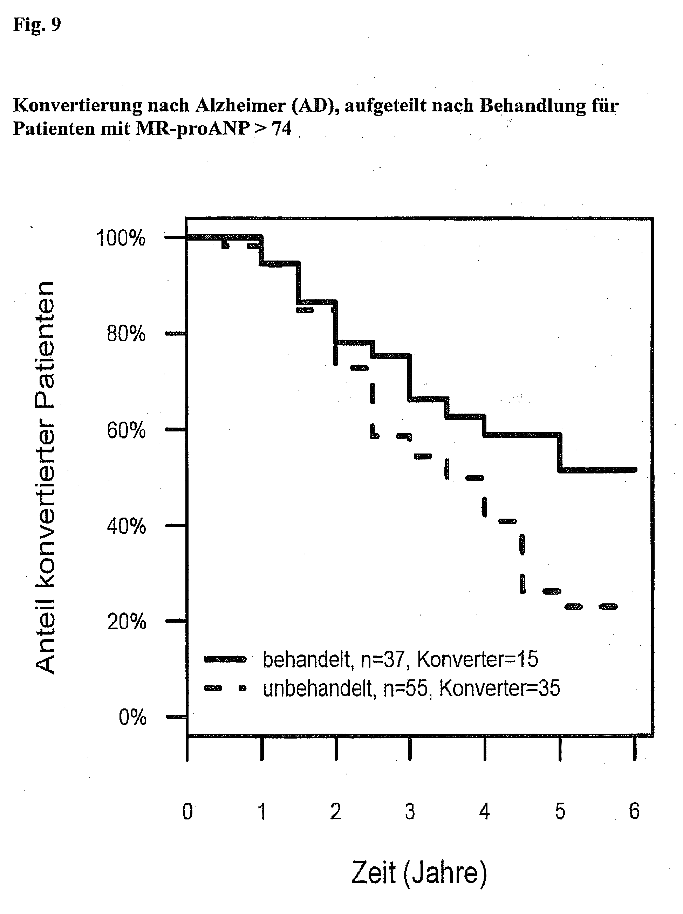

- Figur 9: zeigt die klinisch diagnostizierte Konvertierung nach AD einer Untergruppe von 92 Patienten von den 131 ursprünglichen LKS-Patienten, für die ein MR-proANP-Wert über dem Cut-Off von 74 pmol/l gemessen worden war, getrennt einerseits nach Patienten, die laut ihrer Krankenakte vor Diagnosestellung mit Herz-Kreislauf-Mitteln behandelt worden waren (behandelt), und andererseits nach Patienten, für die keine derartige Vorbehandlung dokumentiert war (unbehandelt).

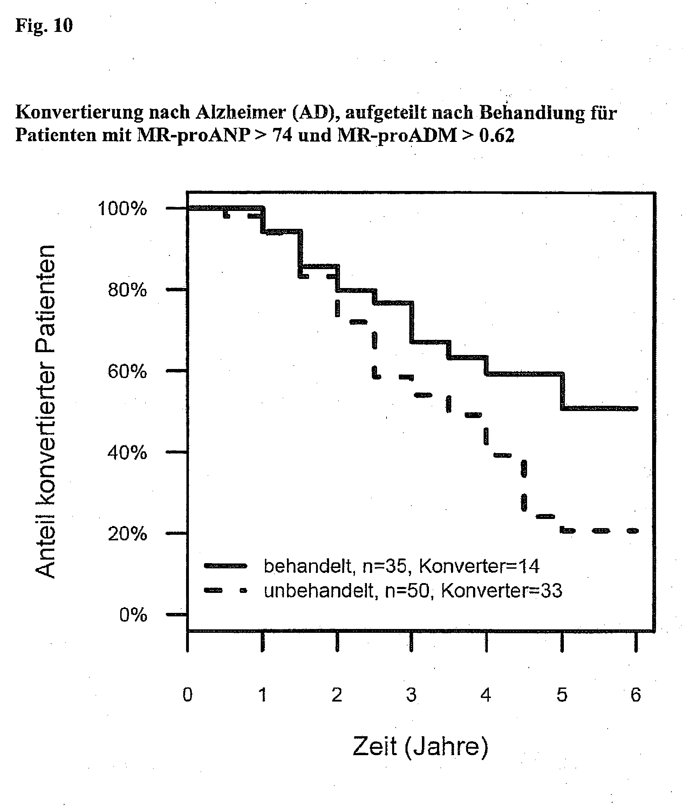

- Figur 10: zeigt die klinisch diagnostizierte Konvertierung nach AD einer Untergruppe derjenigen 85 Patienten von den 131 ursprünglichen LKS-Patienten, für die sowohl ein MR-proANP-Wert über dem Cut-Off von 74 pmol/l als auch ein MR-proADM-Wert über dem Cut-Off von 0,62 nmol/l gemessen worden war, getrennt einerseits nach Patienten, die laut ihrer Krankenakte vor Diagnosestellung mit Herz-Kreislauf-Mitteln behandelt worden waren (behandelt), und andererseits nach Patienten, für die keine derartige Vorbehandlung dokumentiert war (unbehandelt).

### Experimenteller Teil

### Patientenkollektiv:

Von 131 Patienten mit der Diagnose leichte kognitive Störungen (LKS bzw. MCI) und einem Altersmedian von 71,5 Jahren (n=131) wurden bei der ersten Diagnosestellung (t=0) EDTA-Blut-Proben gewonnen, und daraus wurden EDTA-Plasma-Proben als Untersuchungsmaterial für Biomarker-Messungen generiert.

Die Patienten wurden in den folgenden 6 Jahren in 6-Monats-Abständen regelmäßig untersucht. Es wurde jedes Mal festgehalten, ob eine Konversion zu einer klinisch manifesten neurodegenerativen Erkrankung (Alzheimer Demenz, vaskuläre Demenz) eingetreten war.

Alzheimer-Demenz wurde diagnostiziert nach den NINCDS-ADRDA Kriterien (McKhann et al., Neurology 1984; 34: 939-944). MCI wurde diagnostiziert nach den Kriterien von Petersen (Arch Neurol 1999; 56: 303-308).

Im Beobachtungszeitraum entwickelten von den 131 Patienten 75 Patienten (Konverter) eine dokumentierte Neurodegeneration (n=60 Alzheimer Demenz; n=15 vaskuläre Demenz), 56 Patienten (Nicht-Konverter) verblieben im LKS-Status.

Figur 1 zeigt die genannten Befunde für das gesamte Patientenkollektiv in Form eines nur auf der klinischen Diagnose basierenden Kaplan-Meier-Plots.

Tabelle 2 zeigt die Zahlen der LKS-Patienten (Patienten mit einem Risiko zur Entwicklung einer Demenz) und deren klinische Entwicklung im Untersuchungszeitraum, und zwar ausgedrückt als Zahl der Patienten, die eine Konvertierung nach Alzheimer zeigten (AD Konverter), die eine Konvertierung nach einer anderen Demenzform zeigten (Nicht AD Konverter), die keinerlei Konvertierung zeigten (Nicht-Konverter) sowie den prozentualen Anteilen der AD-Konverter, bezogen auf die Zahl der Patienten in der Studie.

**Tabelle 2**

| Konvertierung nach Alzheimer (AD) | | | | | | |
|---|---|---|---|---|---|---|
| | Einschluss | 12 Monate | 24 Monate | 36 Monate | 48 Monate | 60 Monate |
| Patienten in der Studie | 131 | 131 | 131 | 131 | 125 | 106 |
| AD Konverter | 0 | 6 | 27 | 41 | 50 | 57 |
| Nicht AD Konverter | 0 | 3 | 8 | 17 | 17 | 18 |
| Nicht-Konverter | 131 | 122 | 96 | 73 | 58 | 31 |
| Anteil AD Konverter* | 0% | 4,6% | 20,6% | 31,3% | 40,0% | 53,8% |

| | | | | | | |
|---|---|---|---|---|---|---|
| *Berechnet im Bezug auf Patienten in der Studie | | | | | | |

### Biomarker-Messungen / Assaybeschreibung und Auswertung

In den EDTA-Plasma-Proben, die bei Diagnosestellung gewonnen worden waren, wurden mit Assays der Anmelderin die Konzentrationen der Peptid-Biomarker MR-proANP und MR-proADM gemessen.

Die Messung von MR-proANP im Plasma erfolgte mit einem immunoluminometrischen Sandwichassay im Wesentlichen so, wie im experimentellen Teil der o.g. WO 2004/046181 beschrieben wird.

Die Messung von MR-proADM im Plasma erfolgte mit einem immunoluminometrischen Sandwichassay im Wesentlichen so, wie im experimentellen Teil der o.g. WO 2004/090546 beschrieben wird.

Wie in den genannten älteren Patentanmeldungen gezeigt wurde, werden für Gesunde (60 symptomfreie Kontrollpersonen, die weder Symptome kognitiver Störungen zeigten noch an irgendeiner anderen erkennbaren Erkrankung litten, für die bekannt ist, dass bei ihr der jeweilige Biomarker-Analyt erhöht gemessen werden kann), Mediane für die gemessene Konzentrationen wie folgt ermittelt:
MR-proANP = 62,3 pmol/l
MR-proADM = 0,60 nmol/l.

### Auswertungen

Die im bei Diagnosestellung gewonnenen Plasma der 131 LKS-Patienten gemessenen Konzentrationen der Peptid-Biomarker sind in den Figuren 2 (für MR-proADM) bzw. in Figur 3 (für MR-proANP) aufgetragen, und zwar jeweils getrennt nach späteren Konvertern nach AD, Konvertern nach anderen Demenzen (nach vaskulärer Demenz)und Nicht-Konvertern.

Es wurde gefunden, dass sich für beide Biomarker bereits bei der ersten Diagnosestellung die Mediane für die Gruppen der verschiedenen Konverter und der Nicht-Konverter signifikant unterscheiden.

Wie in der nachfolgenden Tabelle 3 zusammenfassend gezeigt wird, sind die Mediane der für spätere Konverter gemessenen Konzentrationswerte für beide Biomarker signifikant erhöht.

**Tabelle 3**

| | MR-proADM | MR-proANP |
|---|---|---|
| Nicht-Konverter | 0,71 nmol/l | 73 pmol/l |
| Nicht AD Konverter | 0,99 nmol/l | 125 pmol/l |
| AD Konverter | 0,83 nmol/l | 113 pmol/l |

Die Ermittlung eines für die weitere Auswertung optimalen Cut-Off-Werts für den jeweiligen Biomarker erfolgte auf der Basis von sogenannten ROC-Plots (nicht gezeigt). Es wurde gefunden, dass die Cut-Off-Werte teilweise nur relativ geringfügig über den für Kontrollpersonen ermittelten Medianen liegen können.

Die Figuren 4 und 5 zeigen die Kaplan-Meier-Plots, die bei Anwendung der obigen Cut-Off-Werte auf das Kollektiv von LKS-Patienten erhalten werden, aufgeteilt nach Patientengruppen mit Konzentrationswerten für den angegebenen Peptid-Bioinarker über dem jeweiligen Cut-Off (größer), und solchen darunter (kleiner).

Wie die Figuren 4 und 5 erkennen lassen, besteht bei Messung von MR-proANP- bzw. MR-proADM-Konzentrationen im Plasma von LKS-Patienten, die bei der ersten Diagnosestellung über den für die genannten Assays ermittelten Cut-Off-Werten liegen, für die jeweiligen Patienten eine erheblich höhere Wahrscheinlichkeit zur späteren Konversion als bei niedrigeren Werten.

Das Risiko der Entwicklung von Neurodegenerationen vom Typ Alzheimer Demenz ist somit mit der Erhöhung der ausgewählten Blutrnarker assoziiert. Von den Patienten, für die MR-proADM Konzentrationen im Plasma unter dem Cut-Off von 0,62 nmol/l MR-proADM gemessen wurden, entwickelten nur 17,4% eine AD-Erkrankung, wohingegen 60,1% der Patienten mit Werten über dem genannten Cut-Off nach AD konvertieren. Das Risiko der Konversion liegt bei Konzentrationen über dem Cut-Off somit etwa 3,45 mal höher.

Analog konvertierten 67,1 % der Patienten mit Werten über dem Cut-Off von 74 pmol/l für MR-proANP und nur 18,2% derjenigen Patienten, für die darunter liegende MR-proANP-Konzentrationen gemessen wurden; das Risiko erhöht sich somit für Patienten mit MR-proANP-Konzentrationswerten über dem Cut-Off um den Faktor von etwa 3,7.

Durch Kombination von Biomarkern lassen sich die Risikoprognosen noch weiter verbessern: Exemplarisch wurden bei der Auswertung der obigen Messergebnisse daher 3 Patientengruppen gebildet: a) Patienten mit beiden Markern unterhalb der gewählten Cut-Off-Werte, b) Patienten mit einem der Marker über dem entsprechenden Cut-Off, und d) Patienten mit beiden Markern über den jeweiligen Cut-Off-Werten. Figur 6 zeigt die bei einer solchen Auswertung erhaltene Kaplan-Meier-Darstellung.

Wie Figur 6 zu entnehmen ist, ergibt sich, wenn man beide Biomarker gemeinsam berücksichtigt, dass Patienten, bei denen bei Diagnosestellung (t=0) beide Biomarker unter den jeweiligen Cut-Off-Werten gefunden werden, mit sehr hoher Wahrscheinlichkeit zur Gruppe der Nicht-Konverter gehören, während dann, wenn beide Biomarker gleichzeitig über den entsprechenden Cut-Off-Werten gefunden werden, für einen solchen Patienten die Wahrscheinlichkeit einer Konversion nach AD innerhalb von 5 Jahren sehr hoch ist.

Das relative Risiko der Entwicklung einer manifesten Neurodegeneration vom Typ AD zwischen der Gruppe "beide Marker unterhalb Cut-Off" (6,8% Konversion) und "beide Marker über Cut-Off" (69% Konversion) erhöht sich auf etwa 11.

### Auswertung unter Berücksichtigung von Vorbehandlungen der Patienten mit Herz-Kreislauf Mitteln

Die gemessenen Biomarker ADM und ANP, deren Freisetzung in die Zirkulation indirekt anhand der MR-proANP- bzw. MR-proADM-Fragmente gemessen wurde, sind bekannt als Herzinsuffizienz-Biomarker. Somit ergibt sich eine starke Assoziation zwischen Herzinsuffizienz-Biomarker-Erhöhungen und der Entwicklung von neurodegenerativen Erkrankungen, vor allem der Alzheimer Demenz. Die Korrelation zeigt sich allerdings auf einem überraschend niedrigen Niveau der Biomarker-Konzentrationen, die innerhalb der für die jeweiligen Marker ermittelten Streuungen der "Normalkonzentrationen" liegen. Es handelt sich somit um eher geringfügige Erhöhungen der betrachteten Marker. Daraus zurück schließend wurde der Schluss gezogen, dass bei Patienten, die später eine Neurodegeneration, insbesondere vom Typ AD, entwickeln, auch eine zwar nur geringe, aber kontinuierliche Verschlechterung der Kreislaufleistungsfahigkeit zu beobachten ist.

Hieraus entwickelte der Erfinder die Hypothese, dass eine Stabilisierung des Kreislaufs von LKS-Patienten mit leicht erhöhten kreislaufrelevanten Peptid-Biomarker-Konzentrationen im Sinne einer Verhinderung oder Verzögerung der Konversion wirksam sein könnte.

Untersucht man jedoch - ausgehend von den nachfolgend obigen Überlegungen - die Entwicklung aller 131 LKS-Patienten bezüglich der Ausbildung einer klinisch manifesten Alzheimer Demenz (AD) unter Berücksichtigung des Aspekts "vor Diagnosestellung mit Herz-Kreislauf-Mitteln vorbehandelt" und "vor Diagnosestellung nicht mit Herz-Kreislauf-Mitteln vorbehandelt", so können keine signifikanten Unterschiede zwischen den beiden Patientengruppen festgestellt werden. Es wird hierzu auf Figur 7 verwiesen.

Die Lage ändert sich jedoch, wenn man nicht alle LKS-Patienten berücksichtigt, sonder nur diejenigen, für die in den obigen Biomarker-Messungen Plasmakonzentrationen für die vermessenen kreislauf-relevanten Biomarkern über den jeweiligen Cut-Off-Werten ermittelt worden.

Untersucht man anhand der dokumentieren Krankengeschichten der LKS-Patienten des untersuchten Patientenkollektivs, wie viele von denjenigen Patienten, die bei Stellung der Diagnose "LKS" in ihrem Plasma Konzentrationswerte für einen oder beide der untersuchten Peptid-Biomarker MR-proANP und/oder MR-proADM über den gewählten Cut-Off-Werten aufwiesen, aus anderen Gründen bereits mit mindestens einem Medikament von den in der obigen Tabelle 1 aufgelisteten Medikamenten vorbehandelt worden waren, und inwieweit sich eine solche Behandlung in den genannten Patientengruppen in den beobachteten AD-Konversionsraten widerspiegelt, findet man deutliche Korrelationen in dem Sinne, dass Patienten, bei denen einer oder beide der vermessenen kreislauf-relevanten Biomarker bei der Stellung der Diagnose LKS erhöht gefunden wurde(n) und die mit Medikamenten der genannten Klasse der Herz-Kreislauf-Medikamente vorbehandelt worden waren, eine signifikant geringere Konversion nach AD zeigten als entsprechende Patienten, die keine entsprechende Vorbehandlung erhalten hatten.

Die nachfolgenden Tabellen 4, 5 und 6 fasst die Ergebnisse einer solchen Auswertung der Patientendaten zusammen, und zwar einmal bei Berücksichtigung nur von Patienten, für die MR-proANP erhöht gefunden worden war (Tabelle 4), für die MR-proADM erhöht gefunden worden war (Tabelle 5), und ferner von Patienten, für die beide Biomarker erhöht gefunden worden waren.

**Tabelle 4**

| Konvertierung nach Alzheimer (AD), MR-proANP > 74 pmol/l | | |
|---|---|---|
| | Behandelt | Unbehandelt |
| Gesamt | 37 | 55 |
| AD Konverter | 15 | 35 |
| Anteil AD Konverter | 41% | 64% |

Konvertierung nach AD unter Behandlung bei Patienten mit MR-proANP>74, p=0,035
Reduktion Konvertierung nach AD bei Behandlung: 35,9%

**Tabelle 5**

| Konvertierung nach Alzheimer (AD), MR-proADM > 0,62 nmol/l | | |
|---|---|---|
| | Behandelt | Unbehandelt |
| Gesamt | 43 | 65 |
| AD Konverter | 16 | 37 |
| Anteil AD Konverter | 37% | 43% |

Konvertierung nach AD unter Behandlung bei Patienten mit MR-proADM >0,62, p=0,051
Reduktion Konvertierung nach AD bei Behandlung: 14,0%

**Tabelle 6**

| Konvertierung nach Alzheimer (AD), MR-proANP > 74 pmol/l und gleichzeitig MR-proADM > 0,62 nmol/l | | |
|---|---|---|
| | Behandelt | Unbehandelt |
| Gesamt | 35 | 50 |
| AD Konverter | 14 | 33 |
| Anteil AD Konverter | 40% | 66% |

Konvertierung nach Alzheimer (AD) bei mit MR-proANP >74 und MR-proADM >0,62, p=0,026
Reduktion Konvertierung nach AD bei Behandlung: 39,4%

Der Inhalt der Tabellen 4, 5 und 6 ist graphisch in den Figuren 8, 9 bzw. 10 in Form von Kaplan-Meier-Plots gezeigt.

Den Tabellen bzw. den zugehörigen Figuren ist zu entnehmen, dass die Konversionsrate nach AD von medikamentös vorbehandelten Patienten, für die bei Diagnosestellung an sich über den festgesetzten Cut-Öff-Werten liegende Plasmakonzentrationen der gemessenen Peptid-Biomarker gefunden wurden, sehr viel niedriger lag, d.h. dass sich weniger klinisch manifeste AD-Erkrankungen entwickelten, als im Falle vergleichbarer Patienten, die jedoch keine vorherige medikamentöse Behandlung erhalten hatten.

Hieraus lassen sich erhebliche Verbesserungen der Prognose von Patienten mit LKS (bzw. MCI), für die (leicht) erhöhte kreislauf-relevante Biomarker gemessen werden können, ableiten, wenn man solche Patienten rechtzeitig mit einem oder mehreren Herz-Kreislauf Medikamenten (blutdrucksenkenden Mitteln; Mitteln zur Behandlung der Herzinsuffizienz) behandelt.

Folglich kann gemäß der vorliegenden Erfindung für LKS-Patienten, bei denen bei einer vorherigen *in vitro* Analyse im Plasma erhöhte Werte für Peptid-Biomarker (ANP; ADM) gefunden wurden, d.h. für an sich eine eher schlechte Prognose besteht, auch direkt eine Behandlung mit Herz-Kreislaüf Medikamenten empfohlen werden, da diese das an sich festgestellte Konversionsrisiko nach AD absenken kann.

Die Erfindung betrifft somit auch eine Methode zur Prognose und Therapie leichter kognitiver Störungen bzw. Verfahren zur Identifizierung von therapiebedürftigen Patienten und zur präventiven Behandlung derartiger Patienten mit leichten kognitiven Störungen.

## Patentansprüche

1. *In vitro* Verfahren zur Risikostratifizierung von Patienten mit leichten kognitiven Störungen zum Zweck der Ermittlung therapiebedürftiger Patienten mit leichten kognitiven Störungen, umfassend
- die Bestimmung mindestens eines kreislauf-relevanten Peptid-Biomarker in einer Probe einer biologischen Flüssigkeit aus dem Kreislauf eines Patienten mit der Diagnose einer leichten kognitive Störung und
- die Zuordnung eines erhöhten Risikos für die Entwicklung einer klinisch manifesten neurodegenerativen Erkrankung zu einer erhöhten Konzentration für den mindestens einen kreislauf-relevanten Peptid-Biomarker, welche über einen biomarkerspezifischen Schwellenwert (Cut-Off) im Bereich üblicher Konzentrationswerte für diesen Biomarkern bei leichten kognitiven Störungen liegt, wobei der kreislaufrelevante Biomarker ausgewählt ist aus den Fragmenten MR-pro-ANP und/oder MR-proADM und wobei der Schwellenwert für MR-ProANP 60 - 90 pmol/l und der Schwellenwert für MR-ProADM 0,5 - 0,7 nmol/l ist, und wobei Patienten mit dem festgestellten höheren Risiko als therapiebedürftig für die Behandlung mit einem Arzneimittel identifiziert werden, welches einen oder mehrere Wirkstoffe von Herz-Kreislauf-Mitteln umfasst, ausgewählt aus der Gruppe umfassend: ANP-Rezeptor-Antagonisten, Adenosin-Rezeptor-Antagonisten, ACE-Hemmer, Angiotensin-II-Rezeptor-Antagonisten, Betablocker und blutdrucksenkende Diuretika und deren Kombinationen, um die Ausbildung klinisch manifester neurodegenerativer Störungen zu verzögern oder zu verhindern oder den Zustand der leichten kognitiven Störung zu bessern oder zu halten.

2. Verfahren gemäß Anspruch 1, wobei der ANP-Rezeptor-Antagonist ein Glucose-Capronsäure-Polymer ist.

3. Verfahren gemäß Anspruch 1, wobei der Adenosin-Rezeptor-Antagonist 1,3-Dipropyl-8-(3-Noradamantyl)Xanthin ist.

4. Verfahren gemäß einem der Ansprüche 1 oder 2, wobei der Wirkstoff ein ANP-Rezeptor-Antagonist ist und der Antagonist verabreicht wird in einer Dosis, bei der mindestens 50% der ANP-Rezeptoren belegt sind.

5. Verfahren gemäß einem der Ansprüche 1 oder 3, wobei der Wirkstoff ein Adenosin-Rezeptor-Antagonist ist und der Antagonist verabreicht wird in einer Dosis, bei der mindestens 50% der Adenosin-Rezeptoren belegt sind.

6. Verfahren gemäß einem der Ansprüche 1 bis 5, wobei die klinisch manifeste neurodegenerativer Störung vom Typ Alzheimer Demenz ist.

7. Verfahren gemäß einem der Ansprüche 1 bis 6, wobei zur Behandlung eine Patientengruppe ausgewählt wird, die aufgrund einer in ihrem Kreislauf messbaren Erhöhung von Kreislauf-relevanten Pcptid-Biomarkern als Patienten mit einem erhöhten Risiko zur Ausbildung einer klinisch manifesten Alzheimer Demenz identifizierbar sind.

8. Verfahren nach einem der Ansprüche 1 bis 7, **dadurch gekennzeichnet, dass** die Probe eine Serum- oder Plasmaprobe ist.

9. Verfahren nach einem der Ansprüche 1 bis 8, **dadurch gekennzeichnet, dass** eine Behandlung mit einem Herz-Kreislauf-Mittel aus der Gruppe der blutdrucksenkenden Mittel empfohlen wird, das ausgewählt ist aus der Gruppe der Hemmer des "Angiotensin Converting Enzyms" (ACE-Hemmer), der Angiotensin-II-Rezeptor-Antagonisten (der "Sartane"), der Betablocker und der zur Blutdrucksenkung eingesetzten Diuretika.

10. Verfahren nach Anspruch 1, **dadurch gekennzeichnet, dass** man die Bestimmung mit einem immundiagnostischen Bestimmungsverfahren durchführt und dass man MR-proADM oder pro-ANP mit einer gegenüber den eigentlichen kreisläufrelevanten Peptiden ADM oder ANP verminderten oder nicht feststellbaren physiologischen Wirkung bestimmt.

## Claims

1. An *in vitro* method for risk stratification of patients with mild cognitive disorders for the purpose of identifying patients with mild cognitive disorders in need of therapy, comprising
- determining in a sample of a biological fluid from the circulation of a patient diagnosed with a mild cognitive disorder at least one circulation-relevant peptide biomarker and
- allocating an increased risk of developing a clinically manifest neurodegenerative disease to an elevated concentration of the at least one circulation-relevant peptide biomarker exceeding a biomarker-specific threshold value (cut-off) set within the range of usual concentration values for this biomarker in the case of mild cognitive disorders, wherein the circulation-relevant biomarker is selected from the MR-proANP and/or MR-proADM fragments and wherein the threshold value for MR-proANP is 60 - 90 pmol/l and for MR-proADM 0.5 - 0.7 nmol/l, and wherein patients with the determined higher risk are identified as those in need of therapy and treatment with a drug comprising one or more active substances of cardiovascular agents selected from the group comprising: ANP receptor antagonists, adenosine receptor antagonists, ACE inhibitors, angiotensin II receptor antagonists, beta blockers and antihypertensive diuretics and combinations thereof, in order to delay or prevent the development of clinically manifest neurodegenerative disorders or to improve or maintain the status of the mild cognitive disorder.

2. The method in accordance with Claim 1, wherein the ANP receptor antagonist is a glucose-caproic acid polymer.

3. The method in accordance with Claim 1, wherein the adenosine receptor antagonist is 1,3-dipropyl-8-(3-noradamantyl)xanthine.

4. The method in accordance with Claim 1 or 2, wherein the active substance is an ANP receptor antagonist and the antagonist is administered in a dose at which at least 50% of the ANP receptors are occupied.

5. The method in accordance with Claim 1 or 3, wherein the active substance is an adenosine receptor antagonist and the antagonist is administered in a dose at which at least 50% of the adenosine receptors are occupied.

6. The method in accordance with any of Claims 1 to 5, wherein the clinically manifest neurodegenerative disorder is of the Alzheimer's dementia type.

7. The method in accordance with any of Claims 1 to 6, wherein for treatment a group of patients is selected who, on the basis of an elevation of circulation-relevant peptide biomarkers measurable in their circulation, can be identified as patients at increased risk of developing a clinically manifest form of Alzheimer's dementia.

8. The method in accordance with any of Claims 1 to 7, **characterized in that** the sample is a serum or plasma sample.

9. The method in accordance with any of Claims 1 to 8, **characterized in that** a treatment is recommended with a cardiovascular agent from the group of antihypertensive agents selected from the group of "Angiotensin Converting Enzyme" inhibitors (ACE inhibitors), angiotensin II receptor antagonists ("sartanes"), beta blockers and diuretics used to lower blood pressure.

10. The method in accordance with Claim 1, **characterized in that** determination is carried out with an immunodiagnostic determination method and that MR-proADM or proANP are determined with a reduced or undetectable physiological effect vis-à-vis the actual circulation-relevant peptides ADM or ANP.

## Revendications

1. Procédé *in vitro* de stratification de risques des patients présentant des troubles cognitifs légers pour identifier les patients ayant des troubles cognitifs légers qui nécessitent un traitement. Le procédé consiste à :
- déterminer dans un échantillon de liquide biologique prélevé du système vasculaire d'un patient chez qui il a été diagnostiqué un trouble cognitif léger au moins un biomarqueur peptidique caractéristique du système vasculaire; et
- attribuer un risque accru de développement d'une maladie neurodégénérative cliniquement manifeste à une concentration élevée du au moins un biomarqueur peptidique caractéristique du système vasculaire excédant une valeur seuil de biomarqueur spécifique (valeur limite) fixée dans la plage des valeurs de concentration habituelles pour ce biomarqueur en cas de troubles cognitifs légers, le biomarqueur peptidique caractéristique du système vasculaire étant choisi parmi les fragments MR-proANP et/ou MR-proADM et la valeur seuil étant 60 - 90 pmol/L pour MR-proANP et 0,5 - 0,7 nmol/L pour MR-proADM, et les patients présentant le risque le plus élevé déterminé étant identifiés comme ceux qui nécessitent une thérapie et un traitement avec un médicament contenant une ou plusieurs substances actives d'agents cardiovasculaires choisis parmi le groupe comprenant : des antagonistes de récepteurs ANP, des antagonistes de récepteurs d'adénosine, des inhibiteurs ECA, des antagonistes de récepteurs d'angiotensine II, des bêta-bloquants et des diurétiques antihypertenseurs et leurs combinaisons, pour retarder ou prévenir le développement de troubles neurodégénératifs cliniquement manifestes ou pour améliorer ou maintenir l'état du trouble cognitif léger.

2. Procédé selon la revendication 1, l'antagoniste de récepteur ANP étant un polymère du glucose et de l'acide capronique.

3. Procédé selon la revendication 1, l'antagoniste de récepteurs d'adénosine étant 1,3-dipropyl-8-(3-noradamantyl)xanthine.

4. Procédé selon la revendication 1 ou 2, la substance active étant un antagoniste de récepteurs ANP et l'antagoniste étant administré à une dose à laquelle au moins 50% des récepteurs ANP sont occupés.

5. Procédé selon la revendication 1 ou 3, la substance active étant un antagoniste de récepteurs d'adénosine et l'antagoniste étant administré à une dose à laquelle au moins 50% des récepteurs d'adénosine sont occupés.

6. Procédé selon une quelconque des revendications 1 à 5, le trouble neurodégénératif cliniquement manifeste étant une démence de type Alzheimer.

7. Procédé selon une quelconque des revendications 1 à 6, un groupe de patients étant sélectionnés pour le traitement qui, sur la base d'une élévation de biomarqueurs peptidiques caractéristiques du système vasculaire mesurables dans leur système vasculaire, peuvent être identifiés comme étant des patients qui présentent un risque accru de développement d'une forme cliniquement manifeste de la démence d'Alzheimer.

8. Procédé selon une quelconque des revendications 1 à 7, **caractérisé en ce que** l'échantillon est un échantillon de sérum ou de plasma.

9. Procédé selon une quelconque des revendications 1 à 8, **caractérisé en ce que** le traitement recommandé est un agent cardiovasculaire parmi le groupe d'agents antihypertenseurs sélectionnés parmi le groupe composé d'inhibiteurs d'« enzymes de conversion de l'angiotensine » (inhibiteurs ECA), d'antagonistes de récepteurs d'angiotensine II (« sartanes »), de bêta-bloquants et de diurétiques utilisés pour baisser la tension artérielle.

10. Procédé selon la revendication 1, **caractérisé en ce que** ladite détermination est effectuée à l'aide d'un procédé de détermination d'immunodiagnostic, et **en ce que** le MR-proADM ou le proANP sont déterminés avec un effet physiologique réduit ou indétectable par rapport aux peptides ADM ou ANP réelles pertinentes au système vasculaire.
